# EUROPEAN PATENT SPECIFICATION

(11) **EP 0 949 931 B1**
(45) Date of publication and mention of the grant of the patent: **27.08.2008**
(21) Application number: 97954087.9
(22) Date of filing: 08.12.1997
(51) Int. Cl.: A61K 45/06, A61K 38/20

(54) **COMBINATION THERAPY USING AN IL-1 INHIBITOR FOR TREATING IL-1 MEDIATED DISEASES**
IL-1-INHIBITOR IN KOMBINATIONSTHERAPIE ZUR BEHANDLUNG IL-1-VERMITTELTER KRANKHEITEN
THERAPIE COMBINEE UTILISANT UN INHIBITEUR DE L'IL-1 POUR TRAITER LES MALADIES INDUITES PAR L'IL-1

(30) Priority: 06.12.1996 US 32790 P; 23.01.1997 US 36353 P; 07.02.1997 US 39311 P; 09.07.1997 US 52025 P
(43) Date of publication of application: 20.10.1999
(73) Proprietor: Amgen Inc.,, Thousand Oaks, California 91320-1799 (US)
(72) Inventor: BENDELE, Alison, M., Nederland, CO 80466 (US); SENNELLO, Regina, M., Boulder, CO 80302 (US)
(74) Representative: Grünecker, Kinkeldey, Stockmair & Schwanhäusser Anwaltssozietät
(86) International application number: PCT/US1997/022720
(87) International publication number: WO 1998/024477

(56) References cited:
- WO-A-96/20729
- AU-B- 649 245
- M. BRODY ET AL.: "MECHANISM OF ACTION OF METHOTREXATE: EXPERIMENTAL EVIDENCE THAT METHOTREXATE BLOCKS THE BINDING OF INTERLEUKIN 1 BETA TO THE INTERLEUKIN 1 RECEPTOR ON TARGET CELLS." EUROPEAN JOURNAL OF CLINICAL CHEMISTRY AND CLINICAL BIOCHEMISTRY, vol. 31, no. 10, October 1993, BERLIN, DE, pages 667-674, XP002062344 cited in the application
- M. SEITZ ET AL.: "METHOTREXATE ACTION IN RHEUMATOID ARTHRITIS: STIMULATION OF CYTOKINE INHIBITOR AND INHIBITION OF CHEMOKINE PRODUCTION BY PERIPHERAL BLOOD MONONUCLEAR CELLS." BRITISH JOURNAL OF RHEUMATOLOGY, vol. 34, no. 7, July 1995, LONDON, GB, pages 602-609, XP002062345 cited in the application
- A.M. BENDELE ET AL.: "EFFECTS OF INTERLEUKIN-1 RECEPTOR ANTAGONIST ALONE AND IN COMBINATION WITH METHOTREXATE IN ADJUVANT ARTHRITIC RATS." ARTHRITIS AND RHEUMATISM, vol. 40, no. 9 (SUPPL.), 8 - 12 November 1997, NEW YORK, N.Y., US, page S181 XP002062346

## Description

### Field of the Invention

The present invention relates to the field of IL-1 mediated diseases. More specifically, the present invention relates to combination therapy for the purpose of preventing or treating IL-1 mediated diseases.

### Background of the Invention

Inflammation is the body's defense reaction to injuries such as those caused by mechanical damage, infection or antigenic stimulation. An inflammatory reaction may be expressed pathologically when inflammation is induced by an inappropriate stimulus such as an autoantigen, is expressed in an exaggerated manner or persists well after the removal of the injurious agents. Such inflammatory reaction may include the production of certain cytokines.

While the etiology of inflammation is poorly-understood, considerable information has recently been gained regarding the molecular aspects of inflammation. This research has led to identification of certain cytokines which are believed to figure prominently in the mediation of inflammation. Cytokines are extracellular proteins that modify the behavior of cells, particularly those cells that are in the immediate area of cytokine synthesis and release. One of the most potent inflammatory cytokines yet discovered and a cytokine which is thought to be a key mediator in many diseases and medical conditions is interleukin-1 (IL-1). IL-1, which is manufactured (though not exclusively) by cells of the macrophage/monocyte lineage, may be produced in two forms: IL-1 alpha (IL-1α) and IL-1 beta (IL-1β).

A disease or medical condition is considered to be an "interleukin-1 mediated disease" if the spontaneous or experimental disease or medical condition is associated with elevated levels of IL-1 in bodily fluids or tissue or if cells or tissues taken from the body produce elevated levels of IL-1 in culture. In many cases, such interleukin-1 mediated diseases are also recognized by the following additional two conditions: (1) pathological findings associated with the disease or medical condition can be mimicked experimentally in animals by the administration of IL-1; and (2) the pathology induced in experimental animal models of the disease or medical condition can be inhibited or abolished by treatment with agents which inhibit the action of IL-1. In most interleukin-1 mediated diseases at least two of the three conditions are met, and in many interleukin-1 mediated diseases all three conditions are met.

IL-1 mediated diseases such as rheumatoid arthritis and psoriatic arthritis are chronic joint diseases that afflict and disable, to varying degrees, millions of people worldwide. Rheumatoid arthritis is a disease of articular joints in which the cartilage and bone are slowly eroded away by a proliferative, invasive connective tissue called pannus, which is derived from the synovial membrane. The disease may involve peri-articular structures such as bursae, tendon sheaths and tendons as well as extra-articular tissues such as the subcutis, cardiovascular system, lungs, spleen, lymph nodes, skeletal muscles, nervous system (central and peripheral) and eyes (Silberberg (1985), Anderson's Pathology, Kissane (ed.), II:1828).

It is believed that rheumatoid arthritis results from the presentation of a relevant antigen to an immunogenetically susceptible host. The antigens that could potentially initiate an immune response resulting in rheumatoid arthritis might be endogenous or exogenous. Possible endogenous antigens include collagen, mucopolysaccharides and rheumatoid factors. Exogenous antigens include mycoplasms, mycobacteria, spirochetes and viruses. By-products of the immune reaction inflame the synovium (i.e., prostaglandins and oxygen radicals) and trigger destructive joint changes (i.e., collagenase).

There is a wide spectrum of disease severity, but many patients run a course of intermittent relapses and remissions with an overall pattern of slowly progressive joint destruction and deformity. The clinical manifestations may include symmetrical polyarthritis of peripheral joints with pain, tenderness, swelling and loss of function of affected joints; morning stiffness; and loss of cartilage, erosion of bone matter and subluxation of joints after persistent inflammation. Extra-articular manifestations include rheumatoid nodules, rheumatoid vasculitis, pleuropulmonary inflammations, scleritis, sicca syndrome, Felty's syndrome (splenomegaly and neutropenia), osteoporosis and weight loss (Katz (1985), Am. J. Med., 79:24 and Krane and Simon (1986), Advances in Rheumatology, Synderman (ed.), 70(2):263-284). The clinical manifestations result in a high degree of morbidity resulting in disturbed daily life of the patient.

One generally accepted theory which is used to explain the causal link between IL-1 and arthritis is that IL-1 stimulates various cell types, such as fibroblasts and chondrocytes, to produce and secrete proinflammatory or degradative compounds such as prostaglandin E₂ and metalloproteinases. The involvement of interleukin-1 in arthritis has been implicated by two distinct lines of evidence.

First, increased levels of interleukin-1, and of the mRNA encoding it, have been found in the synovial tissue and fluid of arthritic joints. See, for example, Buchan et al., "Third Annual General Meeting of the British Society for Rheumatology," London, England, November 19-21, 1988, J. Rheumatol., 25(2); Fontana et al. (1982), Rheumatology Int., 2:49-53; and Duff et al. (1988), Monokines and Other Non-Lymphocytic Cytokines, M. Powanda et al. (eds), pp. 387-392 (Alan R. Liss, Inc.).

Second, the administration of interleukin-1 to healthy joint tissue has been shown on numerous occasions to result in the erosion of cartilage and bone. In one experiment, intra-articular injections of IL-1 into rabbits were shown to cause cartilage destruction *in vivo* (Pettipher et al. (1986), Proc. Nat'l Acad. Sci. U.S.A., 83:8749-8753). In other studies, IL-1 was shown to cause the degradation of both cartilage and bone in tissue explants (Saklatavala et al. (1987), Development of Diseases of Cartilage and Bone Matrix, Sen and Thornhill (eds.), pp. 291-298 (Alan R. Liss, Inc.) and Stashenko et al. (1987), The American Association of Immunologists, 183:1464-1468). Moreover, a recent preliminary human clinical trial in rheumatoid arthritis with an inhibitor of IL-1 has shown promising results (Bresnihan, et al. (1996), Arthritis and Rheumatism, 39(9):S73; and Watt et al. (1996), Arthritis and Rheumatism, 39(9):S123).

Brody et al., Eur. J. Clim. Chem. Clim. Biochem. 31: 667 - 74 disclose the central role of interleukin 1 in inflammatory processes and the induction of the immune response. They also disclose the role of methotrexate in therapy as an IL-1 inhibitor.

The authors conducted experiments using phycoerythrin-labeled IL-1β and concluded that the presence of MTX decreased binding of IL-1β to IL-1 receptor. However, no experiments were conducted demonstrating that MTX usually binds to IL-1 receptor; for example, the authors did not perform an experiment using labeled MTX. Brody *et al*. thus never indicated that MTX is a receptor-binding antagonist like IL-1ra. Furthermore, IL-1ra also blocks IL-α from binding to the IL-1 receptor, and Brody *et al*. never alleged an effect on IL-1 a. More important, the rheumatology community did not adopt the theory of Brody *et al*. as an explanation of the clinical benefits of MTX as indicated by the references below.

Furst (1996) Journal of Rheumatology 23(44):86"90, lists nine separate mechanisms of action for MTX (Furst, page 87, Table 2).

Crilly et al. (1995) Journal of Rheumatology. 22(2):224-226, found that MTX reduced IL-6 and soluble IL-2 receptor; they suggest that the IL-6 effect may result from a reduction in DL-1, which directly contradicts Brody *et al*.'s assumption that a reduction in IL-1 production is not involdved. (Cf. Crilly *et al*. at page 225; Brody *et al*. at page 668).

Seitz et al. (1995) British Society for Rheumatology. 34.602-609, conclude from their experiments that MTX stimulates production of cytokine inhibitors while also inhitiing production and release of inflammatory cytokines, including IL-1 (Seitz *et al*. at page 602; see also Cutolo et al. (2001) Annual Rheumatology, 60:729-735; again, a direct contradiction of Brody et al's assumption.

Cronstein *et al.* (1996) (copy enclosed) propose two biochemical mechanisms of MTX that lead to release of adenosine; their theory attributes MTX's antirheumatic effects to the anti-inflammatory activity of the released adenosine, with not mention of IL-1 inhibition. In their review article, Cronstein *et al.* indirectly criticize the findings of Brody *et al*. (D2).

In summary, MTX action was unclear in the art. Brody et al. and others discovered a large diversity of mechanisms and effects for MTX action in inflammatory diseases.

It is an object of the present invention to provide therapeutic compositions for the treatment of inflammatory conditions of a joint. This and other objects of the present invention will become apparent from the description hereinafter.

### Summary of the Invention

The present invention relates to combination medicament for preventing and treating IL-1 mediated diseases in a patient. The present invention specifically relates to combination medicaments using an IL-1 inhibitor for preventing and treating IL-1 mediated diseases, including rheumatic diseases, and the systemic inflammation and body weight loss associated therewith. The type of treatment herein referred to is intended for mammals, including humans.

### Brief Description of the Figures

Numerous aspects and advantages of the present invention will become apparent upon review of the figures, wherein:
Figure 1 depicts a nucleic acid sequence (SEQ ID NO:1) encoding encoding Arg¹-Glu¹⁵³, mature recombinant human IL-1ra (rhuIL-lra). Also depicted is the amino acid sequence (SEQ ID NO:2) of rhuIL-1ra, with the initial amino acid being Mₙ wherein n equals 0 or 1.
Figure 2 depicts the effects of rhuIL-1ra alone, methotrexate alone and the combination of rhuIL-1ra and methotrexate on joint diameter in the adjuvant arthritic rats in Example 1.
Figure 3 depicts the effects of rhuIL-1ra alone, methotrexate alone and the combination of rhuIL-1ra and methotrexate on final paw weights (index of arthritis), splenomegaly (index of systemic inflammation) and body weight change in the adjuvant arthritic rats in Example 1.
Figure 4 depicts the final analysis (inhibition at termination) of the effects of rhuIL-1ra alone, methotrexate alone and the combination of rhuIL-1ra and methotrexate on joint diameter in the adjuvant arthritic rats in Example 2.
Figure 5 depicts the effects of rhuIL-1ra alone, methotrexate alone and the combination of rhuIL-1ra and methotrexate on final paw weights (index of arthritis), splenomegaly (index of systemic inflammation) and body weight change in the adjuvant arthritic rats in Example 2.

### Detailed Description of the Invention

The compositions of the invention are for administering to an animal afflicted with an IL-1 mediated disease an effective amount of an IL-1 inhibitor in combination with methotrexate. The preferred animal subject is human.

Interleukin-1 inhibitors may be from any protein capable of specifically preventing activation of cellular receptors to IL-1. Classes of interleukin-1 inhibitors include: interleukin-1 receptor antagonists such as IL-1ra, as described below; anti-IL-1 receptor monoclonal antibodies (e.g., EP 623674), reference; IL-1 binding proteins such as soluble IL-1 receptors (e.g., U.S.P. 5,492,888, U.S.P. 5,488,032, and U.S.P. 5,464,937, U.S.P. 5,319,071, and U.S.P. 5,180,812, anti-IL-1 monoclonal antibodies (e.g., WO 9501997, WO 9402627, WO 9006371, U.S.P. 4,935,343, EP 364778, EP 267611 and EP 220063, ); IL-1 receptor accessory proteins (e.g., WO 96/23067,), and other compounds and proteins which block in vivo synthesis or extracellular release of IL-1.

Interleukin-1 receptor antagonist (IL-1ra) is a human protein that acts as a natural inhibitor of interleukin-1 and which is a member of the IL-1 family member which includes IL-1α and IL-1β. Preferred receptor antagonists, as well as methods of making and using thereof, are described in U.S. Patent No. 5,075,222; WO 91/08285; WO 91/17184; AU 9173636; WO 92/16221; WO93/21946; WO 94/06457; WO 94/21275; FR 2706772; WO 94/21235; DE 4219626, WO 94/20517; WO 96/22793 and WO 97/28828.

The proteins include glycosylated as well as non-glycosylated IL-1 receptor antagonists.

Specifically, three useful forms of IL-1ra and variants thereof are disclosed and described in the 5,075,222 patent. The first of these, IL-1raα, is characterized as a 22-23 kD molecule on SDS-PAGE with an approximate isoelectric point of 4.8, eluting from a Mono Q FPLC column at around 52 mM NaCl in Tris buffer, pH 7.6. The second, IL-1raβ, is characterized as a 22-23 kD protein, eluting from a Mono Q column at 48 mM NaCl. Both IL-1raα and IL-1raβ are glycosylated. The third, IL-1rax, is characterized as a 20 kD protein, eluting from a Mono Q column at 48 mM NaCl, and is non-glycosylated. 5,075,222 patent also discloses methods for isolating the genes responsible for coding the inhibitors, cloning the gene in suitable vectors and cell types, and expressing the gene to produce the inhibitors

For purposes of the present invention, IL-1ra and modified forms of IL-1ra in which amino acids of IL-1ra have been (1) deleted from ("deletion variants"), (2) inserted into ("addition variants") or (3) substituted for ("substitution variants") are collectively termed "IL-lra protein(s)". [Unless otherwise indicated, amino acid numbering for molecules - described herein shall correspond to that presented for the mature form of molecule (i.e., minus the signal sequence), as depicted by amino acids Arg¹-Glu¹⁵² of SEQ ID NO:2, with the initial MET in each such sequence being residue number "0".]

It will be appreciated by those skilled in the art that many combinations of deletions, insertions and substitutions (individually or collectively "variant(s)") can be made within the amino acid sequences of IL-1ra, provided that the resulting molecule is biologically active (e.g., possesses the ability to inhibit IL-1).

An IL-ra variant(s) may be rapidly screened to assess its physical properties. It will be appreciated that such variant(s) will demonstrate similar IL-1 inhibiting properties, but not necessarily all of the same properties and not necessarily to the same degree as IL-1ra.

There are two principal variables in the construction of amino acid sequence variant(s): the location of the mutation site and the nature of the mutation. In designing a variant(s), the location of each mutation site and the nature of each mutation will depend on the biochemical characteristic(s) to be modified. Each mutation site can be modified individually or in series, e.g., by (1) deleting the target amino acid residue, (2) inserting one or more amino acid residues adjacent to the located site or (3) substituting first with conservative amino acid choices and, depending upon the results achieved, then with-more radical selections.

Amino acid sequence deletions generally range from about 1 to 30 amino acid residues, preferably from about 1 to 20 amino acid residues, more preferably from about 1 to 10 amino acid residues and most preferably from about 1 to 5 contiguous residues. Amino-terminal, carboxy-terminal and internal intrasequence deletions are contemplated. Deletions within the IL-1ra amino acid sequence may be made, for example, in regions of low homology with the sequences of other members of the IL-1 family. Deletions within the IL-1ra amino acid sequence in areas of substantial homology with the sequences of other members of the IL-1 family will be more likely to significantly modify the biological activity.

An amino acid sequence addition may include insertions of an amino- and/or carboxyl-terminal fusion ranging in length from one residue to one hundred or more residues, as well as internal intrasequence insertions of single or multiple amino acid residues. Internal additions may range generally from about 1 to 20 amino acid residues, preferably from about 1 to 10 amino acid residues, more preferably from about 1 to 5 amino acid residues, and most preferably from about 1 to 3 amino acid residues. Additions within the amino acid sequences of IL-1ra may be made in regions of low homology with the sequences of other members of the IL-1 family. Additions within the amino acid sequence of IL-1ra in areas of substantial homology with the sequences of other members of the IL-1 family will be more likely to significantly modify the biological activity. Additions preferably include amino acid sequences derived from the sequences of IL-1 family members.

An amino-terminus addition is contemplated to include the addition of a methionine (for example, as an artifact of the direct expression in bacterial recombinant cell culture). A further example of an amino-terminal addition includes the fusion of a signal sequence to the amino-terminus of IL-1ra in order to facilitate the secretion of protein from recombinant host cells. Such signal sequences generally will be obtained from and thus be homologous to the intended host cell species. For prokaryotic host cells that do not recognize the native signal sequence of IL-1ra, the signal sequence may be substituted by a prokaryotic signal sequence selected, for example, from the group of the alkaline phosphatase, penicillinase or heat-stable enterotoxin II leader sequences. For expression in yeast cells, each polypeptide may have a signal sequence selected, for example, from the group of the yeast invertase, alpha factor or acid phosphatase leader sequences. In mammalian cell expression the native signal sequence of IL-1ra (U.S. 5,075,222) is satisfactory, although other mammalian signal sequences may be suitable, for example sequences derived from other IL-1 family members.

An example of an amino- or a carboxy-terminus addition includes chimeric proteins comprising the amino-terminal or carboxy-terminal fusion of an IL-1ra protein(s) with all or part of the constant domain of the heavy or light chain of human immunoglobulin (individually or collectively, ("IL-1ra Fc(s)"). Such chimeric polypeptides are preferred wherein the immunoglobulin portion of each comprises all of the domains except the first domain of the constant region of the heavy chain of human immunoglobulin such as IgG (e.g., IgG1 or IgG3), IgA, IgM or IgE. A skilled artisan will appreciate that any amino acid of the immunoglobulin portion can be deleted or substituted with one or more amino acids, or one or more amino acids can be added as long as the IL-1ra protein(s) portion still inhibits IL-1 and the immunoglobulin portion shows one or more of its characteristic properties.

Another group of variant(s) is amino acid substitution variant(s) of the amino acid sequence of IL-1ra. These are variant(s) wherein at least one amino acid residue in IL-1ra is removed and a different residue inserted in its place. Substitution variant(s) include allelic variant(s), which are characterized by naturally-occurring nucleotide sequence changes in the species population that may or may not result in an amino acid change. One skilled in the art can use any information known about the binding or active site of the polypeptide in the selection of possible mutation sites. Exemplary substitution variant(s) are taught in WO 91/17184, WO 92/16221 and WO 96/09323.

One method for identifying amino acid residues or regions for mutagenesis of a protein is called "alanine scanning mutagenesis", as described by Cunningham and Wells (1989), Science, 244:1081-1085, In this method, an amino acid residue or group of target residues of a protein is identified (e.g., charged residues such as Arg, Asp, His, Lys and Glu) and replaced by a neutral or negatively-charged amino acid (most preferably alanine or polyalanine) to affect the interaction of the amino acids with the surrounding aqueous environment in or outside the cell. Those domains/residues demonstrating functional sensitivity to the substitutions are then refined by introducing additional or alternate residues at the sites of substitution. Thus, the site for introducing an amino acid sequence modification is predetermined. To optimize the performance of a mutation at a given site, alanine scanning or random mutagenesis may be conducted and the variant(s) may be screened for the optimal combination of desired activity and degree of activity. The receptor binding sites in IL-1ra have been mapped by extensive site-directed mutagenesis (Evans et al. (1994), The Journal of Biological Chemistry, 270(19):11477-11483.

The sites of greatest interest for substitutional mutagenesis include sites in which particular amino acids found within IL-ra are substantially different from other various species or other members of the IL-1 family in terms of side-chain bulk, charge and/or hydrophobicity. Other sites of interest include those in which particular residues of IL-1ra are identical among other species or other IL-1 family members, as such positions are generally important for the biological activity of a protein. A skilled artisan will appreciate that initially these sites should be modified by substitution in a relatively conservative manner.

Such conservative substitutions are shown in Table 1 under the heading of "Preferred Substitutions". If such substitutions result in a change in biological activity, then more substantial changes (Exemplary Substitutions) may be introduced and/or other additions/deletions may be made and the resulting polypeptides screened.

**TABLE 1: Amino Acid Substitutions**

| Original Residue | Preferred Substitutions | Exemplary Substitutions |
|---|---|---|
| Ala (A) | Val | Val; Leu; Ile |
| Arg (R) | Lys | Lys; Gln; Asn |
| Asn (N) | Gln | Gln; His; Lys; |
| | | Arg |
| Asp (D) | Glu | Glu |
| Cys (C) | Ser | Ser |
| Gln (Q) | Asn | Asn |
| Glu (E) | Asp | Asp |
| Gly (G) | Pro | Pro |
| His (H) | Arg | Asn; Gln; Lys; |
| Ile (I) | Leu | Arg Leu; Val; Met; |
| | | Ala; Phe; norleucine |
| Leu (L) | Ile | norleucine; |
| | | Ile; Val; Met; Ala; Phe |
| Lys (K) | Arg | Arg; Gln; Asn |
| Met (M) | - Leu | Leu; Phe; Ile |
| Phe (F) | Leu | Leu; Val; Ile; |
| | | Ala |
| Pro (P) | Gly | Gly |
| Ser (S) | Thr | Thr |
| Thr (T) | Ser | Ser |
| Trp (W) | Tyr | Tyr |
| Tyr (Y) | Phe | Trp; Phe; Thr; |
| | | Ser |
| Val (V) | Leu | Ile; Leu; Met; |
| | | Phe; Ala; |
| | | norleucine |

In making such changes, the hydropathic index of amino acids may be considered. The importance of the hydropathic amino acid index in conferring interactive biological function on a protein is generally understood in the art (Kyte and Doolittle (1982), J. Mol. Biol., 157:105-131,). It is known that certain amino acids may be substituted for other amino acids having a similar hydropathic index or score and still retain a similar biological activity.

It is also understood in the art that the substitution of like amino acids can be made effectively on the basis of hydrophilicity, particularly where the functionally equivalent protein or peptide thereby created is intended for use in immunological embodiments, as in the present case. U.S. Patent 4,554,101, states that the greatest local average hydrophilicity of a protein, as governed by the hydrophilicity of its adjacent amino acids, correlates with its immunogenicity and antigenicity, i.e., with a biological property of the protein.

U.S. Patent 4,554,101 also teaches the identification and preparation of epitopes from primary amino acid sequences on the basis of hydrophilicity. Through the methods disclosed in U.S. Patent 4,554,101 a skilled artisan would be able to identify epitopes, for example, within the amino acid sequence of IL-1ra. These regions are also referred to as "epitopic core regions". Numerous scientific publications have been devoted to the prediction of secondary structure, and to the identification of epitopes, from analyses of amino acid sequences (Chou and-Fasman (1974), Biochemistry, 13(2):222-245; Chou and Fasman (1974), Biochemistry, 13(2):211-222; Chou and Fasman (1978), Adv. Enzymol. Relat. Areas Mol. Biol., 47:45-148; Chou and Fasman (1978), Ann. Rev. Biochem., 47:251-276 and Chou and Fasman (1979), Biophys. J., 26:367-384. Moreover, computer programs are currently available to assist with predicting antigenic portions and epitopic core regions of proteins. Examples include those programs based upon the Jameson-Wolf analysis (Jameson and Wolf (1988), Comput. Appl. Biosci., 4(1):181-186 and Wolf et al. (1988), Comput. Appl. Biosci., 4(1):187-191, the program PepPlot^{®} (Brutlag et al. (1990), CABS, 6:237-245 and Weinberger et al. (1985), Science, 228:740-742, the ); and other programs for protein tertiary structure prediction (Fetrow and Bryant (1993), EIOTECHNOLOGY, 11:479-483.

In contrast, substantial modifications in the functional and/or chemical characteristics of IL-1ra may be accomplished by selecting substitutions that differ significantly in their effect on maintaining (a) the structure of the polypeptide backbone in the area of the substitution, for example, as a sheet or helical conformation, (b) the relative charge or hydrophobicity of the protein at the target site or (c) the bulk of the side chain. Naturally-occurring residues are divided into groups based on common side chain properties:
1) hydrophobic: norleucine, Met, Ala, Val, Leu, Ile;
2) neutral hydrophilic: Cys, Ser, Thr;
3) acidic: Asp, Glu;
4) basic: Asn, Gln, His, Lys, Arg;
5) aromatic: Trp, Tyr, Phe; and
6) residues that influence chain orientation: Gly, Pro.

Non-conservative substitutions may involve the exchange of a member of one of these groups for another. Such substituted residues may be introduced into regions of IL-1ra that, for example, are homologous with other IL-1 family members or into non-homologous regions of the protein.

A variety of amino acid substitutions or deletions may be made to modify or add N-linked or O-linked glycosylation sites, resulting in a protein with altered glycosylation. The sequence may be modified to add glycosylation sites to or to delete N-linked or O-linked glycosylation sites from IL-1ra. An asparagine-linked glycosylation recognition site comprises a tripeptide sequence which is specifically recognized by appropriate cellular glycosylation enzymes. These tripeptide sequences are either Asn-Xaa-Thr or Asn-Xaa-Ser, where Xaa can be any amino acid other than Pro.

Specific mutations of the sequence of IL-1ra may involve substitution of a non-native amino acid at the amino-terminus, carboxy-terminus or at any site of the protein that is modified by the addition of an N-linked or O-linked carbohydrate. Such modifications may be-of particular utility in the addition of an amino acid (e.g., cysteine), which is advantageous for the linking of a water soluble polymer to form a derivative. For example, WO 92/16221 describes the preparation of IL-1ra muteins, e.g., wherein native residues are changed to cysteine.

In a specific embodiment, a variant polypeptide will preferably be substantially homologous to the amino acid of IL-1ra (SEQ ID NO:2). The term "substantially homologous" as used herein means a degree of homology that is in excess of 80%, preferably in excess of 90%, more preferably in excess of 95% or most preferably even 99%. The percentage of homology as described herein is calculated as the percentage of amino acid residues found in the smaller of the two sequences which align with identical amino acid residues in the sequence being compared when four gaps in a length of 100 amino acids may be introduced to assist in that alignment, as set forth by Dayhoff in Atlas of Protein Sequence and Structure, 5:124 (1972), National Biochemical Research Foundation, Washington, D.C..

Also included within the term "substantially homologous" are variant(s) of IL-1ra which may be isolated by virtue of cross-reactivity with antibodies to the amino acid sequence of SEQ ID NO:2 or whose genes may be isolated through hybridization with the DNA of SEQ ID NO:1 or with segments thereof.

### Polypeptide Derivatives

Chemically-modified derivatives of IL-1ra protein(s) in which the protein is linked to a polymer in order to modify properties of the protein (referred herein as "derivatives") are useful for practicing the present invention. Such derivatives may be prepared by one skilled in the art given the disclosures herein. Conjugates may be prepared using glycosylated, non-glycosylated or de-glycosylated IL-1ra protein(s) and suitable chemical moieties. Typically, non-glycosylated proteins and water soluble polymers will be used.

Water soluble polymers are desirable because the protein to which each is attached will not precipitate in an aqueous environment, such as a physiological environment. Preferably, the polymer will be pharmaceutically acceptable for the preparation of a therapeutic product or composition. One skilled in the art will be able to select the desired polymer based on such considerations as whether the polymer/protein conjugate will be used therapeutically and, if so, the therapeutic profile of the protein (e.g., duration of sustained release; resistance to proteolysis; effects, if any, on dosage; biological activity; ease of handling; degree or lack of antigenicity and other known effects of a water soluble polymer on a therapeutic proteins).

Suitable, clinically acceptable, water soluble polymers include, but are not limited to, polyethylene glycol (PEG), polyethylene glycol propionaldehyde, copolymers of ethylene glycol/propylene glycol, monomethoxy-polyethylene glycol, carboxymethylcellulose, dextran, polyvinyl alcohol (PVA), polyvinyl pyrrolidone, poly-1, 3-dioxolane, poly-1,3,6-trioxane, ethylene/maleic anhydride copolymer, poly (β-amino acids) (either homopolymers or random copolymers), poly(n-vinyl pyrrolidone)polyethylene glycol, polypropylene glycol homopolymers (PPG) and other polyalkylene oxides, polypropylene oxide/ethylene oxide copolymers, polyoxyethylated polyols (POG) (e.g., glycerol) and other polyoxyethylated polyols, polyoxyethylated sorbitol, or polyoxyethylated glucose, colonic acids or other carbohydrate polymers, Ficoll or dextran and mixtures thereof. As used herein, polyethylene glycol is meant to encompass any of the forms that have been used to derivatize other proteins, such as mono-(C1-C10) alkoxy- or aryloxy-polyethylene glycol. Polyethylene glycol propionaldehyde may have advantages in manufacturing due to its stability in water.

The water soluble polymers each may be of any molecular weight and may be branched or unbranched. Generally, the higher the molecular weight or the more branches, the higher the polymer:protein ratio. The water soluble polymers each typically have an average molecular weight of between about 2kDa to about 100kDa (the term "about" indicating that in preparations of a water soluble polymer, some molecules will weigh more, some less, than-the stated molecular weight). The average molecular weight of each water soluble polymer preferably is between about 5kDa and about 40kDa, more preferably between about 10kDa and about 35kDa and most preferably between about 15kDa and about 30kDa.

There are a number of attachment methods available to those skilled in the art, including acylation reactions or alkylation reactions (preferably to generate an amino-terminal chemically modified protein) with a reactive water soluble molecule. See, for example, EP 0 401 384; Malik et al. (1992), Exp. Hematol., 20:1028-1035; Francis (1992), Rocs on Growth Factors, 3(2):4-10, published by Mediscript, Mountain Court, Friern Barnet Lane, London N20 OLD, UK; EP 0 154 316; EP 0 401 384; WO 92/16221; WO 95/34326; WO 95/13312; WO 96/11953; WO 96/19459 and WO 96/19459; and the other publications cited herein that relate to pegylation,

A specific embodiment for practicing the present invention is an unbranched monomethoxy-polyethylene glycol aldehyde molecule having an average molecular weight of either about 20kDa or about 33kDa (e.g., between 30kDa and 35kDa), or a tertiary-butyl polyethylene glycol aldehyde having an average molecular weight of about 33kDa (e.g., between 30kDa and 35kDa) conjugated via reductive alkylation to the IL-1ra protein(s).

The pegylation also may be specifically carried out using water soluble polymers having at least one reactive hydroxy group (e.g. polyethylene glycol). The water soluble polymer can be reacted with an activating group, thereby forming an "activated linker" useful in modifying various proteins. The activated linkers can be monofunctional, bifunctional, or multifunctional.

Activating groups which can be used to link the water soluble polymer to two or more proteins include the following: sulfone, maleimide, sulfhydryl, thiol, triflate, tresylate, azidirine, oxirane and 5-pyridyl. Useful reagents having a reactive sulfone group that can be used in the methods include, without limitation, chlorosulfone, vinylsulfone and divinylsulfone. These PEG derivatives are stable against hydrolysis for extended periods in aqueous environments at pHs of about 11 or less, and can form linkages with molecules to form conjugates which are also hydrolytically stable. Two particularly useful homobifunctional derivatives are PEG-bis-chlorosulfone and PEG-*bis*-vinylsulfone (WO 95/13312).

### Polyvalent Forms

- Polyvalent forms, i.e., molecules comprising more than one active moiety, may be constructed. In one embodiment, the molecule may possess multiple interleukin-1 receptor antagonist sites. Additionally, the molecule may possess at least one - interleukin-1 receptor antagonist site and, depending upon the desired characteristic of polyvalent form, at least one site of another molecule (e.g., an IL-1ra protein(s), and a TNFbp product, as described below).

In one embodiment, the polyvalent form may be constructed, for example, by chemically coupling at least one IL-1ra protein(s) and another moiety with any clinically accepted linker (e.g., a water-soluble polymer). In principle the linker must not impart new immunogenecity nor, by virtue of the new amino acid residues, alter the hydrophobicity and charge balance of the structure which affects its biodistribution and clearance.

The water soluble polymers can be, based on the monomers listed herein, homopolymers, random or block copolymers, terpolymers straight chain or branched, substituted or unsubstituted. The polymer can be of any length or molecular weight, but these characteristics can affect the biological properties. Polymer average molecular weights particularly useful for decreasing clearance rates in pharmaceutical applications are in the range of 2,000 to 35,000 daltons. In addition, the length of the polymer can be varied to optimize or confer the desired biological activity.

The active moieties may be linked using conventional coupling techniques (see WO 92/16221, WO 95/13312 and WO 95/34326,). For example, WO 92/16221 and WO 95/34326 describe the preparation of various dimerized IL-1ra molecules.

Alternatively, a bivalent molecule may consist of two tandem repeats of IL-1ra protein(s) separated by a polypeptide linker region. The design of the polypeptide linkers is similar in design to the insertion of short loop sequences between domains in the *de novo* design of proteins (Mutter (1988), TIBS, 13:260-265 and Regan and DeGrado (1988), Science, 241:976-978,). Several different linker constructs have been assembled and shown to be useful for forming single chain antibodies; the most functional linkers vary in size from 12 to 25 amino acids (amino acids having unreactive side groups, e.g., alanine, serine and glycine) which together constitute a hydrophilic sequence, have a few oppositely charged residues to enhance solubility and are flexible (Whitlow and Filpula (1991), Methods: A Companion to Methods in Enzymology, 2:97-105; and Brigido et al. (1993), J. Immunol., 150:469-479,).

Additionally, the IL-1ra protein(s) may be chemically coupled to biotin, and the resulting conjugate may then be allowed to bind to avidin, resulting in tetravalent avidin/biotin/IL-lra protein(s) molecule. IL-1ra protein(s) may also be covalently coupled to dinitrophenol (DNP) or trinitrophenol (TNP) and the resulting conjugate precipitated with anti-DNP or anti-TNP-IgM to form decameric conjugates.

In yet another embodiment, recombinant fusion proteins may also be produced wherein each recombinant chimeric molecule has an IL-1ra protein(s) sequence amino-terminally or carboxy-terminally fused to all or part of the constant domains, but at least one constant domain, of the heavy or light chain of human immunoglobulin. For example, a chimeric IL-1ra protein(s)/IgG1 (or IgG1/IL-1ra protein(s)) fusion protein may be produced from a light chain-containing chimeric gene: an IL-1ra protein(s)/human kappa light chain chimera (IL-1ra protein(s)/Ck) or a human kappa light chain/IL-1ra protein(s) chimera (ck/IL-1ra protein(s)); or a heavy chain-containing chimeric gene: an IL-1ra protein(s)/human gamma-1 heavy chain chimera (IL-1ra protein(s)/Cg-1) or a human gamma-1 heavy chain/IL-1ra protein chimera (Cg-1/IL-lra protein(s)). Following transcription and translation of a heavy-chain chimeric gene, or of a light chain-containing gene and a heavy-chain chimeric gene, the gene products may be assembled into a single chimeric molecule having an IL-1ra protein(s) displayed bivalently. Additional details relating to the construction of such chimeric molecules are disclosed in United States Patent 5,116,964, WO 89/09622, WO 91/16437 and EP 315062.

In yet a further embodiment, recombinant fusion proteins may also be produced wherein each recombinant chimeric molecule has at least one IL-1ra protein(s), as described herein, and at least a portion of the region 186-401 of osteoprotogerin, as described in European Patent Application No. 96309363.8. Either the IL-1ra protein(s) or the portion of osteoprotogerin may be at the amino-terminus or the carboxy-terminus of the chimeric molecule.

### Synthesis of IL-1ra protein(s)

The production of IL-1ra protein(s) is described in further detail below. Such proteins may be prepared, for example, by recombinant techniques or by *in vitro* chemical synthesis protein.

### Polynucleotides

Based upon the present description and using the universal codon table, one of ordinary skill in the art can readily determine all of the nucleic acid sequences which encode the amino acid sequence of the IL-1ra protein(s).

Recombinant expression techniques conducted in accordance with the descriptions set forth below may be followed to produce each such polynucleotide and to express the encoded protein. For example, by inserting a nucleic acid sequence which encodes an IL-1ra protein(s) into an appropriate vector, one skilled in the art can readily produce large quantities of the desired nucleotide sequence. The sequences can then be used to generate detection probes or amplification primers. Alternatively, a polynucleotide encoding an IL-1ra protein(s) can be inserted into an expression vector. By-introducing the expression vector into an appropriate host, the desired protein may be produced in large amounts.

As further described herein, there are numerous host/vector systems available for the propagation of desired nucleic acid sequences and/or the production of the desired proteins. These include, but are not limited to, plasmid, viral and insertional vectors, and prokaryotic and eukaryotic hosts. One skilled in the art can adapt a host/vector system which is capable of propagating or expressing heterologous DNA to produce or express the sequences of the present invention.

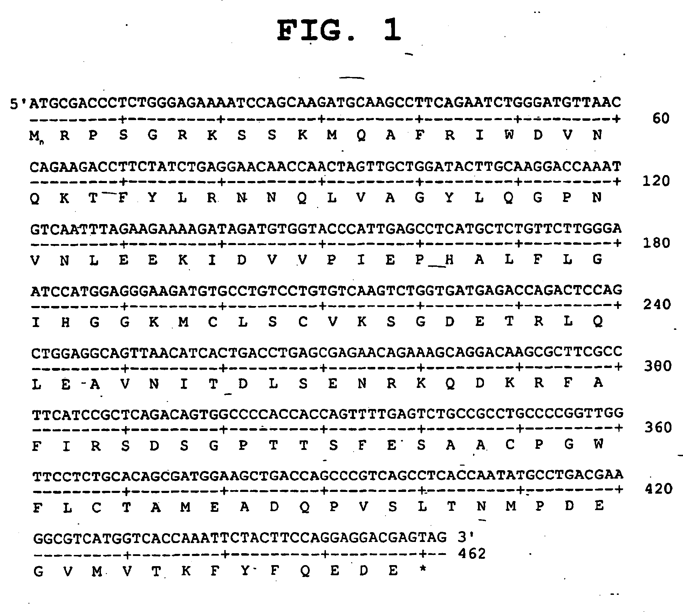

Furthermore, it will be appreciated by those skilled in the art that, in view of the present disclosure, the nucleic acid sequences include the nucleic acid sequence of Figure 1, as well as degenerate nucleic acid sequences thereof, nucleic acid sequences which encode variant(s) of IL-1ra and those nucleic acid sequences which hybridize (under hybridization conditions disclosed in the cDNA library screening section below, or equivalent conditions or more stringent conditions) to complements of the nucleic acid sequence of Figure 1.

### Recombinant Expression

### Preparation of Polynucleotides

A nucleic acid sequence encoding an IL-1ra protein(s) can readily be obtained in a variety of ways including, without limitation, chemical synthesis, cDNA or genomic library screening, expression library screening and/or PCR amplification of cDNA. These methods and others which are useful for isolating such nucleic acid sequences are set forth in Sambrook et al. (1989), Molecular Cloning: A Laboratory Manual, Cold Spring Harbor Laboratory Press, Cold Spring Harbor, NY; by Ausubel et al. (1994), Current Protocols in Molecular Biology, Current Protocols Press; and by Berger and Kimmel (1987), Methods in Enzymology: Guide to Molecular Cloning Techniques, Vol. 152, Academic Press, Inc., San Diego, CA.

Chemical synthesis of a nucleic acid sequence which encodes a desired protein can be accomplished using methods well known in the art, such as those set forth by Engels et al. (1989), Angew. Chem. Int1. Ed., 28:716-734 and Wells et al. (1985), Gene, 34:315,. These methods include, *inter alia*, the phosphotriester, phosphoramidite and H-phosphonate methods of nucleic acid sequence synthesis. Large nucleic acid sequences, for example those larger than about 100 nucleotides in length, can be synthesized as several fragments. The fragments can then be ligated together to form a suitable nucleic acid sequence. A preferred method is polymer-supported synthesis using standard phosphoramidite chemistry.

Alternatively, a suitable nucleic acid sequence may be obtained by screening an appropriate cDNA library (i.e., a library prepared from one or more tissue sources believed to express the protein) or a genomic library (a library prepared from total genomic DNA). The source of the cDNA library is typically a tissue or cell source from any species that is believed to express a desired protein in reasonable quantities. The source of the genomic library may be any tissue or tissues from any mammalian or other species believed to harbor a gene encoding a desired protein.

Hybridization media can be screened for the presence of a DNA encoding a desired protein using one or more nucleic acid probes (oligonucleotides, cDNA or genomic DNA fragments that possess an acceptable level of homology to the cDNA or gene to be cloned) that will hybridize selectively with cDNA(s) or gene(s) present in the library. The probes typically used for such screening encode a small region of DNA sequence from the same or a similar species as the species from which the library is prepared. Alternatively, the probes may be degenerate, as discussed herein.

Hybridization is typically accomplished by annealing an oligonucleotide probe or cDNA to the clones under conditions of stringency that prevent non-specific binding but permit binding of those clones that have a significant level of homology with the probe or primer. Typical hybridization and washing stringency conditions depend in part on the size (i.e., number of nucleotides in length) of the cDNA or oligonucleotide probe and whether the probe is degenerate. The probability of identifying a clone is also considered in designing the hybridization medium (e.g., whether a cDNA or genomic library is being screened).

Where a DNA fragment (such as a cDNA) is used as a probe, typical hybridization conditions include those as set forth in Ausubel et al. (1994), *supra*. After hybridization, the hybridization medium is washed at a suitable stringency, depending on several factors such as probe size, expected homology of probe to clone, the hybridization medium being screened, the number of clones being screened and the like.

Exemplary stringent hybridization conditions are hybridization in 6 x SSC at 62-67°C, followed by washing in 0.1 x SSC at 62-67°C for approximately one hour. Alternatively, exemplary stringent hybridization conditions are hybridization at 45-55% formamide, 6 x SSC at 40-45°C, followed by washing in 0.1 x SSC at 62-67°C for approximately one hour. Also included are DNA sequences which hybridize to the nucleic acid sequences set forth in Figure 1 under relaxed hybridization conditions and which encode an IL-1ra protein(s). Examples of such relaxed stringency hybridization conditions are 6 x SSC at 45-55°C or hybridization with 30-40% formamide at 40-45°C, followed by washing in 1-2 x SSC at 55°C for approximately 30 minutes. See Maniatis et al. (1982), Molecular Cloning (A Laboratory Manual), Cold Spring Harbor Laboratory, pages 387 to 389,

There are also exemplary protocols for stringent washing conditions where oligonucleotide probes are used to screen hybridization media. For example, a first protocol uses 6 X SSC with 0.05 percent sodium pyrophosphate at a temperature of between about 35°C and 63°C, depending on the length of the probe. For example, 14 base probes are washed at 35-40°C, 17 base probes at 45-50°C, 20 base probes at 52-57°C, and 23 base probes at 57-63°C. The temperature can be increased 2-3°C where the background non-specific binding appears high. A second protocol uses tetramethylammonium chloride (TMAC) for washing. One such stringent washing solution is 3 M TMAC, 50 mM Tris-HCl, pH 8.0 and 0.2% SDS.

Another method for obtaining a suitable nucleic acid sequence encoding an IL-1ra protein(s) is the polymerase chain reaction (PCR). In this method, cDNA is prepared from poly(A)+RNA or total RNA using the enzyme reverse transcriptase. Two primers, typically complementary to two separate regions of cDNA (oligonucleotides) encoding the desired protein, are then added to the cDNA along with a polymerase such as *Taq* polymerase and the polymerase amplifies the cDNA region between the two primers.

The oligonucleotide sequences selected as probes or primers should be of adequate length and sufficiently unambiguous so as to minimize the amount of non-specific binding that may occur during screening or PCR amplification. The actual sequence of the probes or primers is usually based on conserved or highly homologous sequences or regions. Optionally, the probes or primers can be fully or partially degenerate, i.e., can contain a mixture of probes/primers, all encoding the same amino acid sequence but using different codons to do so. An alternative to preparing degenerate probes is to place an inosine in some or all of those codon positions that vary by species. The oligonucleotide probes or primers may be prepared by chemical synthesis methods for DNA, as described herein.

### Vectors

DNA encoding the desired proteins may be inserted into vectors for further cloning (amplification of the DNA) or for expression. Suitable vectors are commercially available or may be specifically constructed. The selection or construction of an appropriate vector will depend on (1) whether it is to be used for DNA amplification or for DNA expression, (2) the size of the DNA to be inserted into the vector and (3) the intended host cell to be transformed with the vector.

The vectors each typically involve a nucleic acid sequence which encodes a desired protein operatively linked to one or more of the following expression control or regulatory sequence capable of directing, controlling or otherwise effecting the expression of a desired protein by a selected host cell. Each vector contains various components, depending on its function (amplification of DNA or expression of DNA) and its compatibility with the intended host cell. The vector components generally include, but are not limited to, one or more of the following: a signal sequence, an origin of - replication, one or more selection or marker genes, a promoter, an enhancer element, a transcription termination sequence and the like. These components may be obtained from natural sources or be synthesized by known procedures.

Examples of suitable prokaryotic cloning - vectors include bacteriophages such as lambda derivatives, or plasmids from *E. coli* (e.g. pBR322, col E1, pUC, the F-factor and Bluescript^{®} plasmid derivatives (Stratagene, LaJolla, CA)). Other appropriate expression vectors, of which numerous types are known in the art for the host cells described below, can also be used for this purpose.

### Signal Sequence

The nucleic acid encoding a signal sequence may be inserted 5' of the sequence encoding a desired protein, e.g, it may be a component of a vector or it may be a part of a nucleic acid encoding the desired protein. The nucleic acid encoding the native signal sequence of IL-1ra is known (U.S. Patent No. 5,075,222).

### Origin of Replication

Expression and cloning vectors each generally include a nucleic acid sequence that enables the vector to replicate in one or more selected host cells. In a cloning vector, this sequence is typically one that enables the vector to replicate independently of the host chromosomal DNA and includes an origin of replication or autonomously replicating sequence. Such sequences are well known. The origin of replication from the plasmid pBR322 is suitable for most Gram-negative bacteria, and various origins (e.g., Simian Virus 40 (SV40), polyoma, adenovirus, VSV or BPV) are useful for cloning vectors in mammalian cells. Generally, the origin of replication is not needed for mammalian expression vectors (for example, the SV40 origin is often used only because it contains the early promoter).

### Selection Gene

The expression and cloning vectors each typically contain a selection gene. This gene encodes a "marker" protein necessary for the survival or growth of the transformed host cells when grown in a selective culture media. Host cells that are not transformed with the vector will not contain the selection gene and, therefore, will not survive in the culture media. Typical selection genes encode proteins that (a) confer resistance to antibiotics or other toxins, e.g., ampicillin, neomycin, methotrexate or tetracycline; (b) complement auxotrophic deficiencies or (c) supply critical nutrients not available from the culture media.

Other selection genes may be used to amplify the genes to be-expressed. Amplification is the process wherein genes which are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The cell transformants are placed under selection pressure which only the transformants are uniquely adapted to survive by virtue of the marker being present in the vectors. Selection pressure is imposed by culturing the transformed cells under conditions in which the concentration of selection agent in the media is successively changed, thereby leading to amplification of both the selection gene and the DNA that encodes the desired protein. As a result, increased quantities of the desired protein are synthesized from the amplified DNA.

For example, cells transformed with the DHFR selection gene are first identified by culturing all of the transformants in a culture media that contains methotrexate, a competitive antagonist of DHFR. An appropriate host cell when wild-type DHFR is used is the Chinese hamster ovary cell line deficient in DHFR activity (Urlaub and Chasin (1980), Proc. Natl. Acad. Sci., USA, 77(7):4216-4220,. The transformed cells are then exposed to increased levels of methotrexate. This leads to the synthesis of multiple copies of the DHFR gene and, concomitantly, multiple copies of other DNA present in the expression vector, such as the DNA encoding a desired protein.

### Promoter

Expression and cloning vectors each will typically contain a promoter that is recognized by the host organism and is operably linked to a nucleic acid sequence encoding the desired protein. A promoter is an untranslated sequence located upstream (5') to the start codon of a structural gene (generally within about 100 to 1000 bp) that controls the transcription and translation of a particular nucleic acid sequence. A promoter may be conventionally grouped into one of two classes, inducible promoters and constitutive promoters. An inducible promoter initiates increased levels of transcription from DNA under its control in response to some change in culture conditions, such as the presence or absence of a nutrient or a change in temperature. A large number of promoters, recognized by a variety of potential host cells, are well known. A promoter may be operably linked to DNA encoding a desired protein by removing-the promoter from the source DNA by restriction enzyme digestion and inserting the desired promoter sequence. The native IL-1ra promoter sequence may be used to direct amplification and/or expression of the DNA encoding a desired protein. A heterologous promoter is preferred, however, if it permits greater transcription and higher yields of the expressed protein as compared to the native promoter and if it is compatible with the host cell system that has been selected for use. For example, any one of the native promoter sequences of other IL-1 family members may be used to direct amplification and/or expression of the DNA encoding a desired protein.

Promoters suitable for use with prokaryotic hosts include the beta-lactamase and lactose promoter systems; alkaline phosphatase, a tryptophan (trp) promoter system; a bacterial luminescence (luxR) gene system and hybrid promoters such as the tac promoter. Other known bacterial promoters are also suitable. Their nucleotide sequences have been published, thereby enabling one skilled in the art to ligate them to the desired DNA sequences using linkers or adaptors as needed to supply any required restriction sites.

Suitable promoter sequences for use with yeast hosts are also well known in the art. Suitable - promoters for use with mammalian host cells are well known and include those obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and, most preferably, SV40. Other suitable mammalian promoters include heterologous mammalian promoters, e.g., heat-shock promoters and the actin promoter.

### Enhancer Element

The expression and cloning vectors each will typically contain an enhancer sequence to increase the transcription by higher eukaryotes of a DNA sequence encoding a desired protein. Enhancers are cis-acting elements of DNA, usually from about 10-300 bp in length, that act on the promoter to increase its transcription. Enhancers are relatively orientation and position independent. They have been found 5' and 3' to the transcription unit. Yeast enhancers are advantageously used with yeast promoters. Several enhancer sequences available from mammalian genes are known (e.g., globin, elastase, albumin, alpha-feto-protein and insulin). Additionally, viral enhancers such as the SV40 enhancer, the cytomegalovirus early promoter enhancer, the polyoma enhancer, and adenovirus enhancers are exemplary enhancing elements for the activation of eukaryotic promoters. While an enhancer may be spliced into a vector at a position 5' or 3' to a DNA encoding a desired protein, it is typically located at a site 5' from the promoter.

### Transcription Termination

Expression vectors used in eukaryotic host cells each will typically contain a sequence necessary for the termination of transcription and for stabilizing the mRNA. Such sequences are commonly available from the 5' and occasionally 3' untranslated regions of eukaryotic DNAs or cDNAs. These regions contain nucleotide segments transcribed as polyadenylated fragments in the untranslated portion of the mRNA encoding a desired protein.

### Vector Construction

The construction of a suitable vector containing one or more of the herein-listed components (together with the desired coding sequence) may be accomplished by standard ligation techniques. Isolated plasmids or DNA fragments are cleaved, tailored and religated in the desired order to generate the required vector. To confirm that the correct sequence has been constructed, the ligation mixture may be used to transform *E. coli*, and successful transformants may be selected by known techniques as described herein. Quantities of the vector from-the transformants are then prepared, analyzed by restriction endonuclease digestion and/or sequenced to confirm the presence of the desired construct.

A vector that provides for the transient expression of DNA encoding a desired protein in mammalian cells may also be used. In general, transient expression involves the use of an expression vector that is able to replicate efficiently in a host cell, such that the host cell accumulates many copies of the expression vector and, in turn, synthesizes high levels of the desired protein encoded by the expression vector. Each transient expression system, comprising a suitable expression vector and a host cell, allows for the convenient positive identification of proteins encoded by cloned DNAs as well as for the rapid screening of such proteins for desired biological or physiological properties.

### Host Cells

Any of a variety of recombinant host cells; each of which contains a nucleic acid sequence for use in expressing a desired protein, is also provided by the present invention. Exemplary.prokaryotic and eukaryotic host cells include bacterial, mammalian, fungal, insect, yeast or plant cells.

Prokaryotic host cells include, but are not limited to, eubacteria such as Gram-negative or Gram-positive organisms (e.g., *E. coli* (HB101, DH5a, DH10, and MC1061); *Bacilli* spp. such as *B. subtilis*; *Pseudomonas* spp. such as *P. aeruginosa; Streptomyces* spp.; *Salmonella* spp. such as *S. typhimurium*; or *Serratia* spp. such as *S. marcescans*. In a specific embodiment, a desired protein may be expressed in *E. coli*.

In addition to prokaryotic host cells, IL-1ra protein(s) may be expressed in glycosylated form by any one of a number of suitable host cells derived from multicellular organisms. Such host cells are capable of complex processing and glycosylation activities. In principle, any higher eukaryotic cell culture might be used, whether such culture involves vertebrate or invertebrate cells, including plant and insect cells. Eukaryotic microbes such as filamentous fungi or yeast may be suitable hosts for the expression of a desired protein. *Saccharomyces cerevisiae*, or common baker's yeast, is the most commonly used among lower eukaryotic host microorganisms, but a number of other genera, species and strains are well known and commonly available.

Vertebrate cells may be used, as the propagation of vertebrate cells in culture (tissue culture) is a well-known procedure. Examples of useful mammalian host cell lines include, but are not limited to, monkey kidney CV1 line transformed by SV40 (COS-7), human embryonic kidney line (293 cells or 293 cells subcloned for growth in suspension culture), baby hamster kidney cells and Chinese hamster ovary cells. Other suitable mammalian cell lines include, but are not limited to, HeLa, mouse L-929 cells, 3T3 lines derived from Swiss, Balb-c or NIH mice, and BHK or HaK hamster cell lines. In a specific embodiment, a desired protein may be expressed in COS cells or in baculovirus cells.

A host cell may be transfected and preferably transformed with a desired nucleic acid under appropriate conditions permitting expression of the nucleic acid. The selection of suitable host cells and methods for transformation, culture, amplification, screening and product production and purification are well known in the art (Gething and Sambrook (1981), Nature, 293:620-625 or, alternatively, Kaufman et al. (1985), Mol. Cell. Biol., 5(7):1750-1759, or U.S. Pat. No. 4, 419, 446,.

For example, for mammalian cells without cell walls, the calcium phosphate precipitation method may be used. Electroporation, micro-injection and other known techniques may also be used.

It is also possible that a desired protein may be produced by homologous recombination or with recombinant production methods utilizing control elements introduced into cells already containing DNA encoding a desired protein. Homologous recombination is a technique originally developed for targeting genes to induce or correct mutations in transcriptionally-active genes (Kucherlapati (1989), Prog. in Nucl. Acid Res. and Mol. Biol., 36:301,). The basic technique was developed as a method for introducing specific mutations into specific regions of the mammalian genome (Thomas et al. (1986), Cell, 44:419-428; Thomas and Capecchi (1987), Cell, 51:503-512 and Doetschman et al. (1988), Proc. Natl. Acad. Sci., 85:8583-8587, or to correct specific mutations within defective genes (Doetschman et al. (1987), Nature, 330:576-578,. Exemplary techniques are described in U.S. Patent No. 5,272,071; WO 92/01069; WO 93/03183; WO 94/12650 and WO 94/31560,.

### Culturing the Host Cells

The method for culturing each of the one or more recombinant host cells for production will vary depending upon many factors and considerations; the optimum production procedure for a given situation will be apparent to those skilled in the art through minimal experimentation. Such recombinant host cells are cultured in a suitable media and the expressed protein is then optionally recovered, isolated and purified from the culture media (or from the cell, if expressed intracellularly) by appropriate means known to those skilled in the art.

Specifically, each of the recombinant cells used to produce a desired protein may be cultured in media suitable for inducing promoters, selecting suitable recombinant host cells or amplifying the gene encoding the desired protein. The media may be supplemented as necessary with hormones and/or other growth factors (such as insulin, transferrin or epidermal growth factor), salts (such as sodium chloride, calcium, magnesium and phosphate), buffers (such as HEPES), nucleosides (such as adenosine and thymidine), antibiotics (such as gentamicin), trace elements (defined as inorganic compounds usually present at final concentrations in the micromolar range), and glucose or another energy source. Other supplements may also be included, at appropriate concentrations, as will be appreciated by those skilled in the art. Suitable culture conditions, such as temperature, pH and the like, are also well known to those skilled in the art for use with the selected host cells.

The resulting expression product may then be purified to near homogeneity by using procedures known in the art. Exemplary purification techniques are taught in U.S. Patent No. 5,075,222, and WO 91/08285, .

Preferably, expression product is produced in a substantially pure form. By "substantially pure" is meant IL-1ra, in an unmodified form, has a comparatively high specific activity, preferably in the range of approximately 150,000-500,000 receptor units/mg as defined in Hannum et al. (1990), Nature, 343:336-340 and Eisenberg et al. (1990), Nature, 343:341-346,

### Pharmaceutical Compositions

The present invention encompasses the use of pharmaceutical preparations each containing therapeutically- or prophylacticlly-effective amounts of an IL-1ra protein(s) or a chemical derivative thereof (collectively, "IL-1ra protein product(s)") in admixture with methotrexate and a vehicle. The vehicle preferably includes one or more pharmaceutically and physiologically acceptable formulation materials in admixture with the IL-1ra protein product(s).

The primary solvent in a vehicle may be either aqueous or non-aqueous in nature. In addition, the vehicle may contain pharmaceutically acceptable excipients for modifying or maintaining the pH, preferably between 6.0 and 7.0, more preferably 6.5 (e.g., buffers such as citrates-or phosphates, and amino acids such glycine); viscosity; clarity; color; sterility; stability (e.g., sucrose and sorbitol); odor; rate of dissolution (e.g., solubilizers or solubilizing agents such as alcohols, polyethylene glycols and sodium chloride); rate of release; as well as bulking agents for lyophilized formulation (e.g., mannitol and glycine); surfactants (e.g., polysorbate 20, polysorbate 80, triton and pluronics); antioxidants (e.g., sodium sulfite and sodium hydrogen-sulfite); preservatives (e.g., benzoic acid and salicylic acid); flavoring and diluting agents; emulsifying agents; suspending agents; solvents; fillers; delivery vehicles and other pharmaceutical adjuvants and/or excipients... Other effective administration forms such as parenteral slow-release formulations, inhalant mists, orally-active formulations, or suppositories are also envisioned. The composition may also involve particulate preparations of polymeric compounds such as bulk erosion polymers (e.g., poly(lactic-co-glycolic acid) (PLGA) copolymers, PLGA polymer blends, block copolymers of PEG, and lactic and glycolic acid, poly(cyanoacrylates)); surface erosion polymers (e.g., poly(anhydrides) and poly(ortho esters)); hydrogel esters (e.g., pluronic polyols, poly(vinyl alcohol), poly(vinylpyrrolidone), maleic anhydride-alkyl vinyl ether copolymers, cellulose, hyaluronic acid derivatives, alginate, collagen, gelatin, albumin, and starches and dextrans) and composition systems thereof; or preparations of liposomes or microspheres. Such compositions may influence the physical state, stability, rate of *in vivo* release, and rate of *in vivo* clearance of the present proteins and derivatives. The optimal pharmaceutical formulation for a desired protein will be determined by one skilled in the art depending upon the route of administration and desired dosage. Exemplary pharmaceutical compositions are disclosed in Remington's Pharmaceutical Sciences, 18th Ed. (1990), Mack Publishing Co., Easton, PA 18042, pages 1435-1712; Gombotz and Pettit (1995), Bioconjugate Chem., 6:332-351; WO 94/06457; Leone-Bay, et al. (1995), Journal of Medicinal Chemistry, 38:4263-4269; Haas, et al. (1995), Clinical Immunology and Immunopachology, 76(1):93; WO 94/21275; FR 2706772; WO 94/21235, and WO 97/28828,

Specific sustained release compositions are available from the following suppliers: Depotech (Depofoam^{™}, a multivesicular liposome) and Alkermes (ProLease^{™}, a. PLGA microsphere). Exemplary forms of hyaluronan are disclosed in Peyron and Balazs (1974), Path. Biol., 22(8):731-736; Isdale et al. (1991), J. Drug Dev., 4(2):93-99; Larsen et al. (1993), Journal of Biomedical Materials Research, 27:1129-1134; Namiki, et al. (1982), International Journal of Clinical pharmacology, Therapy and Toxicology, 20(11):501-507; Meyer et al. (1995), Journal of Controlled Release, 35:67-72; Kikuchi et al. (1996), Osteoarthritis and Cartilage, 4:99-110; Sakakibara et al. (1994), Clinical Orthopaedics and Related Research, 299:282-292; Meyers and Brandt (1995), 22(9):1732-1739; Laurent et al. (1995), Acta Orthop Scand, 66(266):116-120; Cascone et al. (1995), Biomaterials, 16(7):569-574; Yerashalmi et al. (1994), Archives of Biochemistry and Biophysics, 313(2):267-273; Bernatchez et al. (1993), Journal of Biomedical materials Research, 27(5):677-681; Tan et al. (1990), Australian Journal of Biotechnology, 4(1):38-43; Gombotz and Pettit (1995), Bioconjugate Chem., 6:332-351; U.S. Patent Nos. 4,582,865, 4,605,691, 4,636,524, 4,713,448, 4,716,154, 4,716,224, 4,772,419, 4,851,521, 4,957,774, 4,863,907, 5,128,326, 5,202,431, 5,336,767, 5,356,883; European Patent Application Nos. 0 507 604 A2 and 0 718 312 A2; and WO 96/05845, Specific hyaluronan compositions are available from the following suppliers: BioMatrix , Inc. Ridgefield, NJ (Synvisc^{™}, a 90:10 mixture of a hylan fluid and hylan gel); Fidia S.p.A., Abano Terme, Italy (Hyalgan^{™}, the sodium salt of a rooster comb-derived hyaluronic acid (-500,000 to ∼700,000 MW)); Kaken Pharmaceutical Co., Ltd., Tokyo, Japan (Artz^{™}, a 1% solution of a rooster-comb derived hyaluronic acid, ∼700,000 MW); Pharmacia AB, Stockholm, Sweden (Healon^{™}, a rooster-comb derived hyaluronic acid, ∼4 x 10' MW); Genzyme Corporation, Cambridge, MA (Surgicoat^{™}, a recombinant hyaluronic acid); Pronova Biopolymer, Inc. Portsmouth, NH (Hyaluronic Acid FCH, a high molecular weight (e.g., ∼1.5-2.2 x 10' MW) hyaluronic acid prepared from cultures of *Streptococcus zooepidemicus*; Sodium Hyaluronate MV, -1.0-1.6 x 10' MW and Sodium Hyaluronate LV, -1.5-2.2 x 10' MW); Calbiochem-Novabiochem AB, Lautelfingen, Switzerland (Hyaluronic Acid, sodium salt (1997 company catalog number 385908) prepared from *Streptococcus sp*.); Intergen Company, Purchase, NY (a rooster-comb derived hyaluronic acid, >1 x 10' MW); Diosynth Inc., Chicago, IL; Amerchol Corp., Edison, NJ and Kyowa Hakko Kogyo Co., Ltd., Tokyo, Japan.

Another specific pharmaceutical formulation is 10 millimolar sodium citrate, 140 millimolar sodium chloride, 0.5 millimolar EDTA, 0.1% polysorbate (w/w) in water, pH6.5 ("citrate buffer formulation"). Another specific formulation is 10 millimolar sodium phosphate, 140 millimolar sodium chloride, between 0.1% (wt/wt) and 0.01% polysorbate (w/w) in water, and, optionally, 0.5 millimolar EDTA, pH6.5 ("phosphate buffer formulation". A still further specific formulation is H-10^{™} hylan fluid, a cross-linked hyaluronic acid (Biomatrix, Inc., Ridgefield, Inc.) to achieve 100 mg/ml IL-1ra and 2% hyaluronic acid (Mr³ 4x10⁶).

Once a pharmaceutical composition for use in the present invention has been formulated, it may be stored in sterile vials as a solution, suspension, gel, emulsion, solid, or a dehydrated or lyophilized powder. Such compositions each may be stored either in a ready-to-use form or in a form (e.g., lyophilized) requiring reconstitution prior to administration.

In a specific embodiment, the present invention may employ kits for producing a single-dose administration unit. The kits may each contain both a first container having a dried protein and a second container having an aqueous formulation. Kits are single and multi-chambered pre-filled syringes; exemplary pre-filled syringes (e.g., liquid syringes, and lyosyringes such as Lyo-Ject®, a dual-chamber pre-filled lyosyringe) are available from Vetter GmbH, Ravensburg, Germany.

The present invention relates to the use of methotrexate and an 1L-1 inhibitor for preparing medicaments for the treatment of certain diseases and medical conditions (which can be characterized as inflammatory diseases) that are mediated by IL-1, as well as the related sequela and symptoms associated therewith. A non-exclusive list of acute and chronic interleukin-1 (IL-1)-mediated diseases includes but is not limited to the following: ALS; Alzheimer's disease; asthma; atherosclerosis; cachexia/anorexia; chronic fatigue syndrome, depression; diabetes (e.g., juvenile onset Type 1 and diabetes mellitus); fever; fibromyelgia or analgesia; glomerulonephritis; graft versus host rejection; hemohorragic shock; hyperalgesia, inflammatory bowel disease; ischemic injury, including cerebral ischemia (e.g., brain injury as a result of trauma, epilepsy, hemorrhage or stroke, each of which may lead to neurodegeneration); lung diseases (e.g., ARDS and pulmonary fibrosis); multiple myeloma; multiple sclerosis; myelogenous (e.g., AML and CML) and other leukemias; myopathies (e.g., muscle protein metabolism, esp. in sepsis); ocular diseases; osteoporosis; Parkinson's disease; pain; pancreatitis; pre-term labor; psoriasis; reperfusion injury; rheumatic diseases (e.g., rheumatoid arthritis, osteoarthritis, juvenile (rheumatoid) arthritis, seronegative polyarthritis, ankylosing spondylitis, Reiter's syndrome and reactive arthritis, psoriatic arthritis, enteropathic arthritis, polymyositis, dermatomyositis, scleroderma, systemic sclerosis, vasculitis, cerebral vasculitis, Lyme disease, staphylococcal-induced ("septic") arthritis, Sjögren's syndrome, rheumatic fever, polychondritis and polymyalgia rheumatica and giant cell arteritis); septic shock; side effects from radiation therapy; temporal mandibular joint disease; tumor metastasis; or an inflammatory condition resulting from strain, sprain, cartilage damage, trauma, orthopedic surgery, infection or other disease processes.

IL-1 inhibitors (e.g., preferably an IL-1ra protein product(s) and more preferably IL-1ra) and methotrexate combination medicaments may be administered to a patient in therapeutically effective amounts for the prevention or treatment of IL-1 mediated diseases, including rheumatic diseases. The term "patient" is intended to encompass animals (e.g., cats, dogs and horses) as well as humans.

Further, the IL-1 inhibitors (e.g., preferably an IL-1ra protein product(s) and more preferably IL-1ra) and methotrexate combination medicaments may be administered via topical, enteral or parenteral administration including, without limitation, infusion, intraarterial, intraarticular, intracapsular, intracardiac, intradermal, intramuscular, intraorbital, intrathecal, intravenous, intraperitoneal, intraspinal, intrasternal injection, intraventricular, subcutaneous, subcuticular, subcapsular, subarachnoid and transtracheal. IL-1 inhibitors (e.g., preferably an IL-1ra protein product(s) and more preferably IL-1ra) may also be administered via oral administration or be administered through mucus membranes, that is, buccally, intranasally, rectally or sublingually for systemic delivery.

It is preferred that IL-1 inhibitors (e.g., preferably an IL-1ra protein product(s) and more preferably IL-1ra) and methotrexate combination medicaments are administered via intraarticular, subcutaneous, intramuscular or intravenous injection. Additionally, IL-1 inhibitors (e.g., preferably an IL-1ra protein product(s) and more preferably IL-1ra) may be administered by continuous infusion (e.g., constant or intermittent implanted or external infusion flow-modulating devices) so as to continuously provide the desired level of IL-1 inhibitors (e.g., preferably an IL-1ra protein product(s) and more preferably IL-1ra) in the blood for the duration of the administration. This is may be accomplished by means of a mini-pump, such as an osmotic mini-pump. In these ways, one can -be assured that the amount of the drugs is maintained at the desired level and one can take blood samples and monitor the amount of drug in the bloodstream. Various pumps are commercially available, for example, from suppliers such as MiniMed Inc., Sylmar, CA (e.g., MT507) and Alza Corp., Palo Alto, CA (e.g. Alzet osmotic pump, model 2MLI).

It is also contemplated that other modes of continuous or near-continuous dosing may be practiced. For example, chemical derivatization may result in sustained release forms of the protein which have the effect of continuous presence in the blood stream, in predictable amounts based on a determined dosage regimen.

Modes of using IL-1 inhibitors (e.g., preferably an IL-1ra protein product(s) and more preferably IL-1ra) for the treatment of IL-1-mediated diseases, including rheumatic diseases (e.g., osteoarthritis, psoriatic arthritis and rheumatoid arthritis), are set forth in Australian Patent Application AU 9173636, in one specific embodiment IL-1 inhibitors (e.g., preferably an IL-1ra protein product(s) and more preferably IL-1ra) may be administered intra-articularly for the treatment of rheumatoid arthritis and osteoarthritis. in another specific embodiment, IL-1 inhibitors (e.g., preferably an IL-1ra protein product(s) and more preferably IL-1ra) may be administered subcutaneously or intramuscularly for the treatment of rheumatoid arthritis, inflammatory bowel disease, multiple sclerosis, multiple myeloma, or myelogenous (e.g., AML and CML) and other leukemias in a still further specific embodiment IL-1 inhibitors (e.g., preferably an IL-1ra protein product(s) and more preferably IL-1ra) may be administered intravenously for the treatment of brain injury as a result of trauma, epilepsy, hemorrhage or stroke, or for the treatment of graft-versus-host disease; or administered intraventricularly for the treatment of brain injury as a result of trauma.

Regardless of the manner of administration, the treatment of IL-1 mediated disease requires a dose or total dose regimen of an IL-1 inhibitor (e.g., preferably an IL-1ra protein product(s) and more preferably IL-1ra) effective to reduce or alleviate symptoms of the disease. Factors in determining the appropriate dosage or total dose regimen can include the disease or condition to be treated or prevented, the severity of the disease, the route of administration, and the age, sex and medical condition of the patient.

Further refinement of the calculations necessary to determine the appropriate dosage for treatment is routinely made by those skilled in the art, especially in light of the dosage information and assays disclosed herein. The frequency of dosing depends on the pharmacokinetic parameters of the IL-1 inhibitor (e.g., preferably an IL-1ra protein product(s) and more preferably IL-1ra) in the formulation used. The IL-1 inhibitors (e.g., preferably an IL-1ra protein product(s) and more preferably IL-1ra) may be administered once, or in cases of severe and prolonged disorders, administered daily in less frequent doses or administered with an initial bolus dose followed by a continuous dose or sustained delivery. It is also contemplated that other modes of continuous or near-continuous dosing may be practiced. For example, chemical derivatization may result in sustained release forms which have the effect of a continuous presence in the bloodstream, in predictable amounts based on a determined dosage or total dosage regimen. The dosage or total dose regimen can also be determined through the use of known assays for determining dosages used in conjunction with appropriate dose-response data.

When administered parenterally, each unit dose, for example, may be up to 200 mg, generally up to 150 mg and more generally up to 100 mg. When administered into an articular cavity, the pharmaceutical composition is preferably administered as a single injection from a 3 to 10 ml syringe containing a dose, for example, of up to 200 mg/ml, generally of up to 150 mg and more generally of up to 100 mg of IL-1 product dissolved in isotonic phosphate buffered saline. The preparation may be administered into an articular cavity at a frequency, for example, of once every 7 to 10 days. In such a manner, administration is continuously conducted, for example, 4 to 5 times while varying the dose if necessary.

As contemplated by the present invention, an IL-1 inhibitor product (e.g., preferably an IL-1ra protein product(s) and more preferably IL-1ra) may be administered as an adjunct to other therapy and also with other pharmaceutical formulations suitable for the indication being treated. An IL-1 inhibitor product (e.g., preferably an IL-1ra protein product(s) and more preferably IL-1ra) and methotrexate may be administered separately or in combination.

In a specific embodiment, the present invention is directed to the use of an IL-1 inhibitor (e.g., preferably an IL-1ra protein product(s) and more preferably IL-1ra) in combination (pretreatment, post-treatment or concurrent treatment) with methotrexate for the treatment of IL-1 mediated diseases, including acute and chronic inflammation such as cachexia/anorexia; chronic fatigue syndrome, depression; diabetes (e.g., juvenile onset Type 1 and diabetes mellitus); fibromyelgia or analgesia; graft versus host rejection; hyperalgesia, inflammatory bowel disease; ischemic injury, including cerebral ischemia (e.g., brain injury as a result of trauma, epilepsy, hemorrhage or stroke, each of which may lead to neurodegeneration); lung diseases (e.g., ARDS and pulmonary fibrosis); multiple sclerosis, ocular diseases; pain; pancreatitis, reperfusion injury; rheumatic diseases (e.g., rheumatoid arthritis, osteoarthritis, juvenile (rheumatoid) arthritis, seronegative polyarthritis, ankylosing spondylitis, Reiter's syndrome and reactive arthritis, psoriatic arthritis, enteropathic arthritis, polymyositis, dermatomyositis, scleroderma, systemic sclerosis, vasculitis, cerebral vasculitis, Lyme disease, staphylococcal-induced ("septic") arthritis, Sjögren's syndrome, rheumatic fever, polychondritis and polymyalgia rheumatica and giant cell arteritis); septic shock; side effects from-radiation therapy; temporal mandibular joint disease; tumor metastasis; or an inflammatory condition resulting from strain, sprain, cartilage damage, trauma, orthopedic surgery, infection or other disease processes.

Present treatment of IL-1-mediated diseases, including acute and chronic inflammation such as rheumatic diseases includes the use of first line drugs for control of pain and inflammation classified as non-steroidal, anti-inflammatory drugs (NSAIDs). Secondary treatments include corticosteroids, slow acting antirheumatic drugs (SAARDs) or disease modifying (DM) drugs. Information regarding the following compounds can be found in The Merck Manual of Diagnosis and Therapy, Sixteenth Edition, Merck, Sharp & Dohme Research Laboratories, Merck & Co., Rahway, NJ (1992) and in Pharmaprojects, PJB Publications Ltd.

In a specific embodiment, the present invention is directed to the use of an IL-1 inhibitor (e.g., preferably an IL-1ra protein product (s) and more preferably IL-1ra) and methotrexate for the treatment of IL-1 mediated diseases, including acute and chronic inflammation such as rheumatic diseases; and graft versus host disease.

In a more specific embodiment, the present invention is directed to the use of an IL-1 inhibitor (e.g., preferably an IL-1ra protein product(s) and more preferably IL-1ra) in combination (pretreatment, post-treatment or concurrent treatment) with methotrexate .

In a specific preferred embodiment, the method comprises the administration (e.g., intra-articular, subcutaneous or intramuscular) of an IL-1 inhibitor (e.g., preferably an IL-1ra protein product(s) and more preferably IL-1ra or IL-1ra Fc, optionally formulated with a controlled release polymer (e.g., a dextran or hyaluronan), the citrate buffer formulation or the phosphate buffer formulation) in combination (pretreatment, post-treatment or concurrent treatment) with methotrexate for the treatment of IL-1-mediated diseases, including acute and chronic inflammation such as cachexia/anorexia; chronic fatigue syndrome, depression; diabetes (e.g., juvenile onset Type 1 and diabetes mellitus); fibromyelgia or analgesia; graft versus host rejection; hyperalgesia, inflammatory bowel disease; ischemic injury, including cerebral ischemia (e.g., brain injury as a result of trauma, epilepsy, hemorrhage or stroke, each of which may lead to neurodegeneration); lung diseases (e.g., ARDS and pulmonary fibrosis); multiple sclerosis, ocular diseases; pain; pancreatitis, reperfusion injury; rheumatic diseases (e.g., rheumatoid arthritis, osteoarthritis, juvenile (rheumatoid) arthritis, seronegative polyarthritis, ankylosing spondylitis, Reiter's syndrome and reactive arthritis, psoriatic arthritis, enteropathic arthritis, polymyositis, dermatomyositis, scleroderma, systemic sclerosis, vasculitis, cerebral vasculitis, Lyme disease, staphylococcal-induced ("septic") arthritis, Sjögren's syndrome, rheumatic fever, polychondritis and polymyalgia rheumatica and giant cell arteritis); septic shock; side effects from radiation therapy; temporal mandibular joint disease; tumor metastasis; or an inflammatory condition resulting from strain, sprain, cartilage damage, trauma, orthopedic surgery, infection or other disease processes.

In a specific preferred embodiment, the method comprises the administration (e.g., intra-articular, subcutaneous or intramuscular) of an IL-1 inhibitor (e.g., preferably an IL-1ra protein product(s) and more preferably IL-1ra or IL-1ra Fc, optionally formulated with a controlled release polymer (e.g., a dextran or hyaluronan), the citrate buffer formulation or the phosphate buffer formulation) in combination (pretreatment, post-treatment or concurrent treatment) with methotrexate to treat arthritis (e.g., osteoarthritis, psoriatic arthritis and/or rheumatoid arthritis) and the symptoms associated therewith.

The surprising and unexpected result disclosed herein is the ability of IL-1ra and methotrexate to act synergistically in the treatment of various symptoms associated with IL-1 mediated diseases, including acute and chronic inflammatory conditions. "Synergistically" is used herein to refer to a situation where the benefit conveyed by the joint administration of an IL-1 inhibitor (e.g., preferably an IL-1ra protein product(s) and more preferably IL-1ra) and methotrexate is greater than the algebraic sum of the effects resulting from the separate administration of components of the combination. As shown in the experiments below, in the adjuvant arthritis model the combination of rhuIL-1ra and methotrexate treatment is synergistic with respect to treating systemic inflammation (i.e., splenomegaly) and weight loss associated with rheumatoid arthritis. Thus, the combined rhuIL-1ra and methotrexate treatment has the advantage of achieving the same result with a lower dose or less frequent administration of methotrexate, thereby reducing any toxic effect and potentially the advantage of persisting activity even after the treatment has terminated.

Methotrexate is an anti-metabolite and immunosuppressive drug. Methotrexate is an effective anti-inflammatory agent with utility in the treatment of severe and disabling psoriasis and rheumatoid arthritis (Hoffmeister (1983), The American Journal of Medicine, 30:69-73 and Jaffe (1988), Arthritis and Rheumatism, 31:299). Methotrexate is N-[4-[(2,4-diamino-6-pteridinyl)methylamino]benzoyl]-L-glutamic acid and has the structural formula:

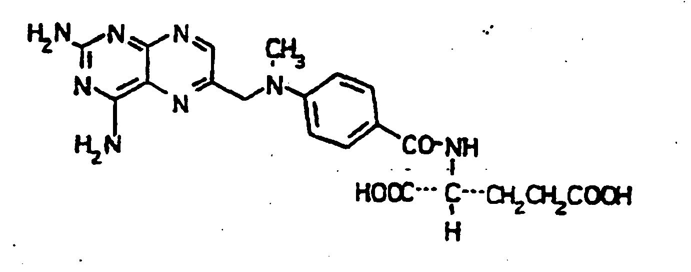

The following references describe the preparation of methotrexate (Seeger et al. (1949), J. Am. Chem. Soc., 71:1753; the metabolism of methotrexate (Freeman (1958), J. Pharmacol. Exp. Ther, 122:154 and Henderson et al. (1965), Cancer Res., 25:1008); the toxicity of methotrexate (Condit et al. (1960), Cancer, 13:222-249; the pharmacokinetic models of methotrexate (Bischoff et al. (1970), J. Pharm, Sci., 59:149); the metabolism and pharmacokinetics of methotrexate (Evans (1980), Appl. Pharmacokinet., Williams et al. (eds.), pp. 518-548 (Appl. Ther., Inc.); the clinical pharmacology of methotrexate (Bertino (1981), Cancer Chemother., 3:359-375 and Jolivet et al. (1983), N. Eng. J. Med., 309:1094-1104); and the clinical experience of methotrexate in rheumatoid arthritis (Weinblatt et al. (1985), N. Eng. J. Med., 312:818-822; Furst (1985), J. Rheumatol., 12(12):1-14; Williams et al. (1985), Arthritis Rheum., 28:721-730 and Seitz et al. (1995), British Journal of Rheumatology, 34:602-609). Additionally, numerous patents have been issued disclosing active agent methotrexate and methods for synthesizing methotrexate or potential intermediates in the synthesis of methotrexate: U.S. Patent Nos. 2,512,572, 3,892,801, 3,989,703, 4,057,548, 4,067,867, 4,079,056, 4,080,325, 4,136,101, 4,224,446, 4,306,064, 4,374,987, 4,421,913 and 4,767,859.

The mechanism of action of methotrexate is poorly understood, however various activities of this drug have been demonstrated which likely contribute to its efficacy (Segal et al. (1990), Seminars in Arthritis and Rheumatism, 20:190-198). The following mechanisms of action for methotrexate have been postulated: inhibition of folate-dependent pathways and protein metabolism (Morgan et al. (1987), Arthritis and Rheumatism, 30:1348-1356); inhibition of neutrophil migration into arthritic joints (Van de Kerkhof et al. (1985), British Journal of Dermatology, 113:251-255; Ternowitz et al. (1987), Journal of Investigative Dermatology, 89:192-196 and Sperling (1992), Arthritis and Rheumatism, 35:376-384); IL-6 inhibitory activity (Segal (1991), Arthritis and Rheumatism, 34(2):146-152) and the local specific anti-proliferative effect on cells involved in arthritis (Rodenhuis et al. (1987), Arthritis and Rheumatism, 30:369-374). Methotrexate has been shown to block the interleukin-1 beta/interleukin-1 receptor pathway (Brody et al. (1993), European Journal of Clinical Chemistry and Clinical Biochemistry, 31(10):667-674); however, although methotrexate may inhibit the proliferative effects of IL-1 and decrease monocyte IL-1 production in the short term in certain patients, this effect is not sustained and is unlikely to explain the-long-term efficacy of methotrexate (Barrera et al. (1996), Seminars in Arthritis and Rheumatism, 25(4):234-253).

Methotrexate may be administered orally, intraperitoneally, subcutaneously or intravenously. Oral administration is preferred. The following is an example of the procedure the combined administration of an IL-1ra protein product(s) (e.g., IL-1ra) and methotrexate treatment to treat a human patient. The patient takes a tablet or capsule of methotrexate three times a week, at a total weekly dose of 5 to 50 mg/patient/week. The patient also is injected intravenously with an IL-1ra protein product(s) (e.g., IL-1ra) at a daily dose of 50 to 150 mg. It will be appreciated by those skilled in the art that the doses presented herein are the preferred doses. The starting dose of the particular compound(s) used is reduced for a patient who exhibits adverse reaction, or the drug used in combination with the compound(s) can be changed or reduced, e.g., depending on the different formulations, routes, dose schedules, and/or other variables known to those skilled in the art such as the individual patient's tolerance of the drug, its efficacy and toxicity.

Preferably, the patient is treated with a weekly starting dose of methotrexate at between 5 mg and 7.5 mg (orally or intramuscularly) and a daily dose of an IL-1ra protein product(s) (e.g., IL-1ra) at between 50 mg and 150 mg (intravenously). Thedosage of methotrexate is increased by 5 mg every 2 to 3 weeks. The maximum dosage level is determined at a point at which the patient shows improvement, which is generally preferably less than about 25 mg of - methotrexate per week, more preferably between 5 to 25 mg of methotrexate per week. The patient may then be evaluated by physical examination and extensive laboratory testing. The tests include evaluation for toxicity. Additional laboratory monitoring in the case of methotrexate preferably includes a complete blood cell count every 2 weeks for the first 3 months and then monthly thereafter. Additional precautions preferably include monthly assessments of levels of serum albumin, alanine amino transferase, bilirubin, creatinine and blood urea nitrogen. Monthly urinalysis is also preferred.

The above is by way of example and does not preclude other treatments to be used concurrently with these anti-inflammatory compounds that are known by those skilled in the art or that could be arrived at by those skilled in the art using the guidelines set forth in this specification.

### Examples

Standard methods for many of the procedures described in the following examples, or suitable alternative procedures, are provided in widely recognized manuals of molecular biology such as, for example, Sambrook et al., Molecular Cloning, Second Edition, Cold Spring Harbor Laboratory Press (1987) and Ausabel et al., Current Protocols in Molecular Biology, Greene Publishing Associates/Wiley Interscience, New York (1990). All chemicals were either analytical grade or USP grade.

### Example 1

An animal model of rheumatoid arthritis induced by an adjuvant was used to investigate the combination therapy of an IL-1 inhibitor and methotrexate. Male Lewis rats (Charles River, Portage, MI) (5 per group) weighing at least 200g were cannulated with jugular catheters and allowed to recover for several days. They were then placed in infusion cages and acclimated for a week prior to initiating adjuvant injections.

On day 0, all treated rats were injected with 100µl of Freunds Complete Adjuvant (Sigma Chemical Co., St. Louis, MO) to which a synthetic adjuvant, N,N-diroctyldecyldecyl-N', N-bis(2-hydroxy-ethyl) propanediamine, 75 mg/ml, was added. On days 0-14 methotrexate in 1% carboxymethylcellulose (Sigma) treatment was started and orally administered (0.06 mg/kg). On day 8, treatment with E. coli-derived human recombinant IL-1 receptor antagonist (prepared generally in accordance with the teachings of U.S.Patent No. 5,075,222, rhuIL-1ra) formulated in a pharmaceutical composition (10 millimolar sodium citrate, 140 millimolar sodium chloride, 0.5 millimolar EDTA, 0.1% polysorbate (w/w) in water, pH6.5) was administered by continuous IV infusion (5mg/kg/hr) to one group of rats being treated with both Freunds Complete Adjuvant and methotrexate and to another group of rats being treated with Freunds Complete Adjuvant alone.

Body weights were taken on day 0 and every other day from day 8 to termination on day 15. Caliper measurements and clinical scoring were done on day 8 and every other day until termination. At this time the animals' body, paw and spleen weights were determined.

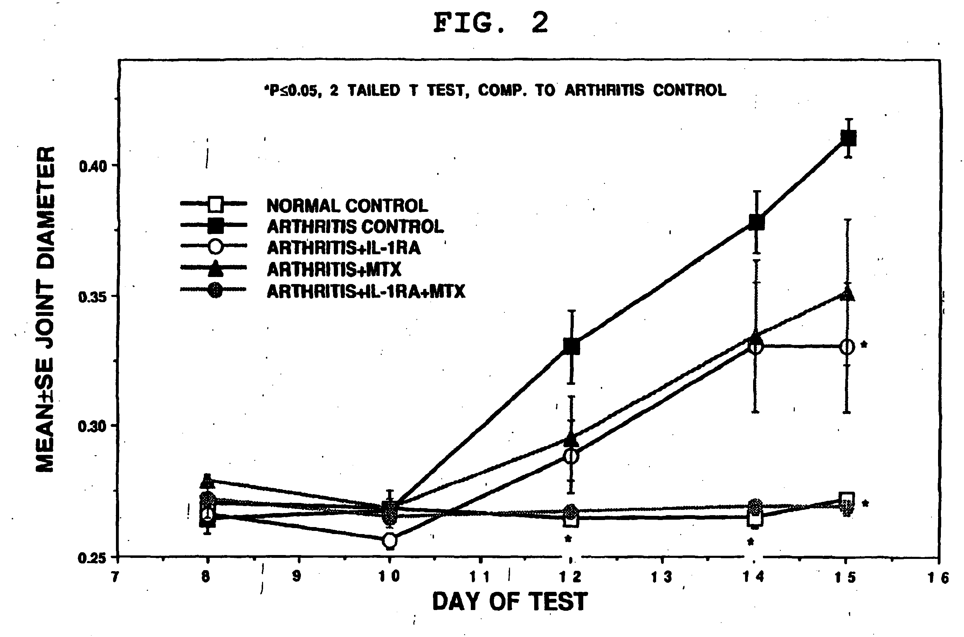

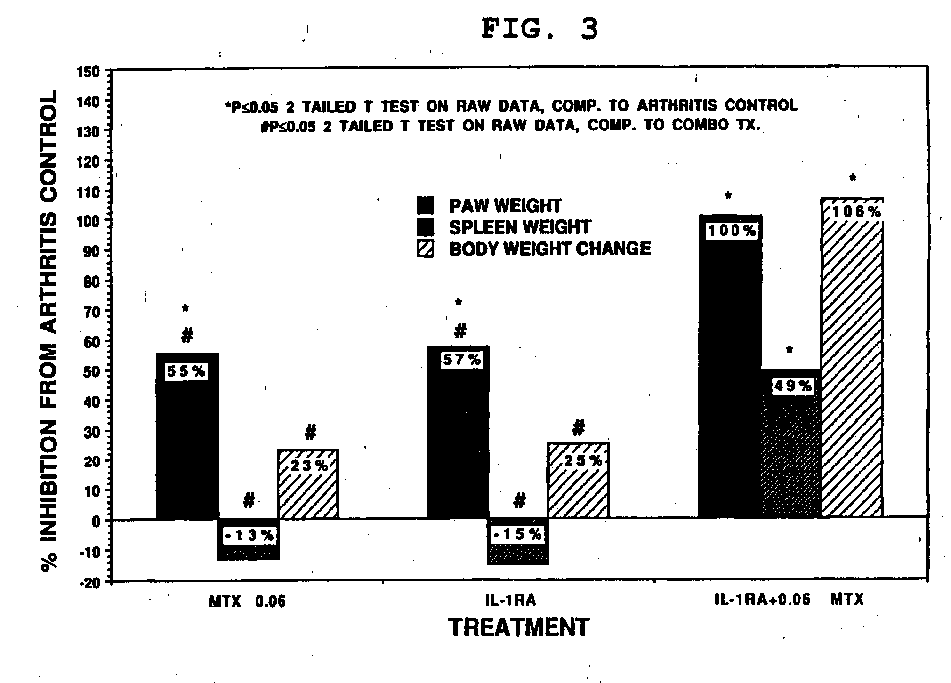

As seen in Figures 2 and 3, rats treated with rhuIL-1ra alone exhibited a 57% inhibition of arthritis (paw swelling), no significant benefit on splenomegaly and 25% inhibition of body weight change. Rats treated with methotrexate had 55% inhibition of paw swelling, no inhibition of spleen weight and 23% inhibition of body weight change. The combination therapy provided 100% inhibition of paw swelling, 49% inhibition of splenomegaly and 106% inhibition of body weight change.

### Example 2

Male Lewis rats (Charles River, Portage, MI) (5-7/group) weighing at least 200g were cannulated with jugular catheters and allowed to recover for several days. They were then placed in infusion cages and acclimated for at least one week prior to initiating adjuvant injections.

On day-0,- all rats were injected with 100µl of Freunds Complete Adjuvant (Sigma Chemical Co., St. Louis, MO) to which a synthetic adjuvant, N,N-dioctyldecyldecyl-N', N-bis(2-hydroxy-ethyl) propanediamine, 75 mg/ml, was added. On days 0-14, methotrexate in 1% carboxymethylcellulose (Sigma) treatment was started and orally administered (0.06 mg/kg). On day 8, treatment with rhuIL-1ra formulated in a pharmaceutical composition (10 millimolar sodium citrate, 140 millimolar sodium chloride, 0.5 millimolar EDTA, 0.1% polysorbate (w/w) in water, pH6.5) by continuous IV infusion (5 mg/kg/hr) was started. Body weights were taken on day 0 and every other day from day 8 to termination on day 15. Caliper measurements and clinical scoring were done on day 8 and every other day until termination. At this time the animals' body, paw and spleen weights were determined.

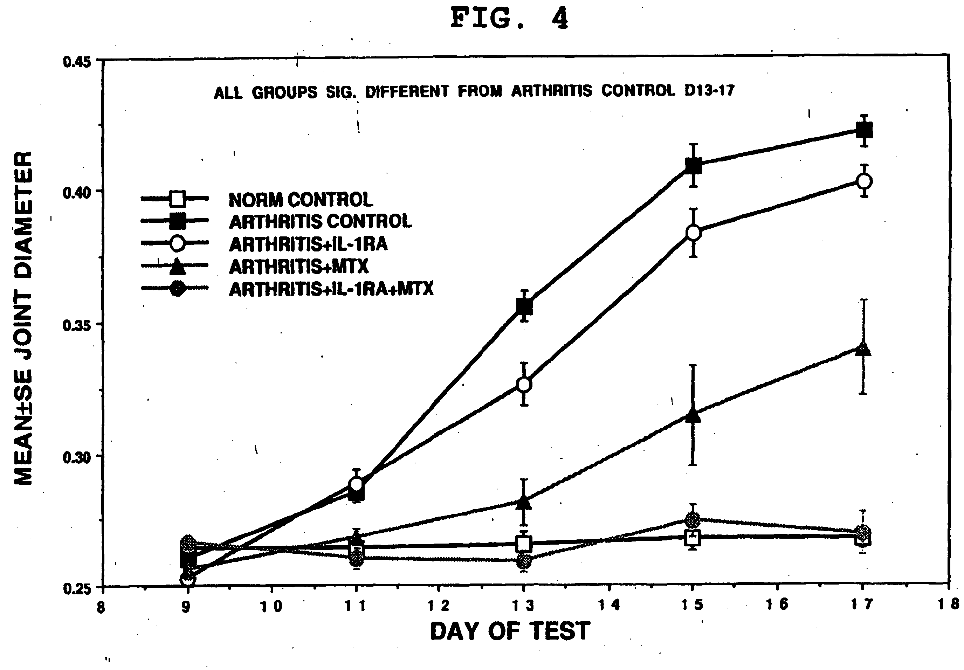

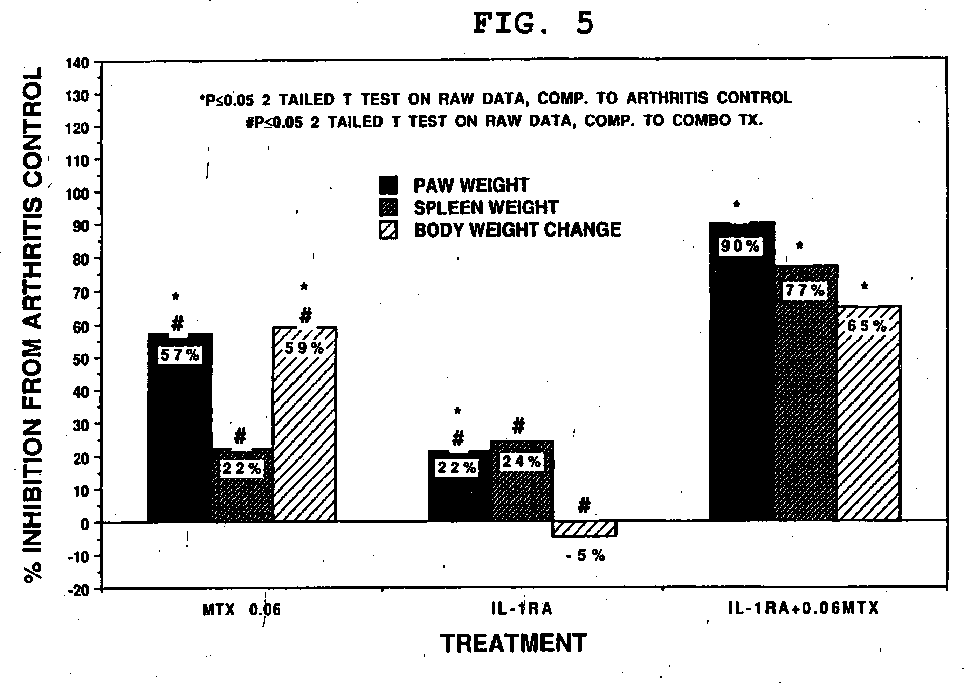

As seen in Figures 4 and 5, rats treated with rhuIL-1ra alone had 22% inhibition of paw swelling, 24% inhibition of splenomegaly, and no inhibition of body weight change. Rats treated with methotrexate had 57% inhibition of paw swelling, 22% inhibition of splenomegaly and 59% inhibition of body weight change. The combination of rhuIL-1ra and methotrexate resulted in 90% inhibition of paw inflammation, 77% inhibition of splenomegaly and 65% inhibition of the body weight change associated with arthritis/systemic inflammation.

### Example 3

Male Lewis rats (Charles River, Portage,-MI) (5-7/group) weighing at least 250-300g were cannulated in the subcutis of the dorsal back and allowed to recover for several days. They were then placed in infusion cages and acclimated for at least 4 days prior to initiating adjuvant injections.

On day-0, all rats were injected with 100µl of Freunds Complete Adjuvant (Sigma Chemical Co., St. Louis, MO) to which a synthetic adjuvant, N,N-dioctyldecyldecyl-N', N-bis(2-hydroxy-ethyl) propanediamine, 75 mg/ml, was added. On days 0-14, methotrexate in 1% carboxymethylcellulose (Sigma) treatment was started and orally administered (0.048, 0.06 or 0.075 mg/kg). On day 8, treatment with rhuIL-1ra formulated in a pharmaceutical composition (10 millimolar sodium citrate, 140 millimolar sodium chloride, 0.5 millimolar EDTA, 0.1% polysorbate (w/w) in water, pH6.5) by continuous subcutaneous infusion (5 mg/ml/hr; 0.1 ml/hr) was started. Body weights were taken on day 0 and every other day from day 8 to termination on day 15. Caliper measurements and clinical scoring were done on day 8 and every other day until termination. At this time the animals' body, paw and spleen weights were determined.

Continuous subcutaneous infusion of rhuIL-1ra at the rate of 5mg/ml/hr, days 8-15 in adjuvant arthritic rats resulted in 6% inhibition of final paw weight. Treatment of adjuvant arthritic rats with daily oral doses of methotrexate (0.075, 0.060 or 0.048 mg/kg) on days 0-14, resulted in 47, 27 or 0% (respectively) inhibition of final paw weight. Concurrent treatment with rhuIL-1ra and methotrexate at these same doses increased the inhibition of final paw weight to 84, 44 or 21%. Therefore, statistically significant clinical benefit was seen when methotrexate doses were 0.075 or 0.06 mg/kg. In the case of combination therapy, inhibition of paw swelling was significantly greater than that occurring with either rhuIL-1ra or methotrexate alone when the dose of methotrexate was 0.075 mg/kg. Analysis of paw swelling by sequential caliper measurement of ankle joints revealed inhibition of arthritis when the data were analyzed as area under the curve. Rats treated with rhuIL-1ra alone had 6% inhibition of swelling while those given 0.075 mg/kg methotrexate had a 45% decrease in ankle joint diameter over time. In contrast, those given the combination therapy had 78% inhibition of arthritis. Statistically significant combination benefit on area under the curve inhibition of ankle diameter was seen in rats given 0.048 but not 0.06 mg/kg methotrexate.

Histologic evaluation of ankle joints from rats treated with rhuIL-1ra demonstrated 8% inhibition of inflammation and 53% inhibition of bone resorption. Treatment with methotrexate resulted in dose responsive significant inhibition of these parameters at 0.075 (44% inhibition of inflammation and 58% inhibition of bone resorption) or 0.06 (26% inhibition of inflammation and 55% inhibition of bone resorption) but not 0.048 mg/kg (8% inhibition of inflammation and 11% inhibition of bone resorption). Combination benefit was most striking in rats given the 0.075 mg/kg dose of methotrexate in combination with rhuIL-1ra. In these rats, inflammation was inhibited by 85% and bone resorption was decreased by 97%. These differences were significantly increased over those seen in either treatment alone for both parameters. Similar combination benefit was also seen at 0.06 mg/kg dose of methotrexate in combination with rhuIL-1ra (44% inhibition of inflammation and 84% inhibition of bone resorption) and with 0.048 mg/kg dose of methotrexate in combination with rhuIL-1ra (23% inhibition of inflammation and 68% inhibition of bone resorption).

### Example 4

Male Lewis rats (Charles River, Portage, MI) (5-7/group) weighing at least 250-300g were cannulated in the subcutis of the dorsal back and allowed to recover for several days. They were then placed in infusion cages and acclimated for at least 4 days prior to initiating adjuvant injections.

On day-0, all rats were injected with 100µl of Freunds Complete Adjuvant (Sigma Chemical Co., St. Louis, MO) to which a synthetic adjuvant, N,N-dioctyldecyldecyl-N', N-bis(2-hydroxy-ethyl) propanediamine, 75 mg/ml, was added. On days 0-14, methotrexate in 1% carboxymethylcellulose (Sigma) treatment was started and orally administered (0.048, 0.06 or 0.075 mg/kg). On day 8, treatment with rhuIL-1ra formulated in a pharmaceutical composition (10 millimolar sodium citrate, 140 millimolar sodium chloride, 0.5 millimolar EDTA, 0.1% polysorbate (w/w) in water, pH6.5) by continuous IV infusion (5 mg/kg/hr) was started. Body weights were taken on day 0 and every other day from day 8 to termination on day 15. Caliper measurements and clinical scoring were done on day 8 and every other day until termination. At this time the animals' body, paw and spleen weights were determined.

Continuous subcutaneous infusion of rhuIL-1ra at the rate of 5mg/kg/hr, days 8-15 in adjuvant arthritic rats resulted in 6% inhibition of final paw weight. Treatment of adjuvant arthritic rats with daily oral doses of methotrexate (0.075, 0.060 or 0.048 mg/kg) on days 0-14, resulted in 47, 27 or 0% (respectively) inhibition of final paw weight. Concurrent treatment with rhuIL-1ra and methotrexate at these same doses increased the inhibition of final paw weight to 84, 44 or 21%. Therefore, statistically significant clinical benefit was seen when methotrexate doses were 0.075 or 0.06 mg/kg. In the case of combination therapy, inhibition of paw swelling was significantly greater than that occurring with either rhuIL-1ra or methotrexate alone when the dose of methotrexate was 0.075 mg/kg. Analysis of paw swelling by sequential caliper measurement of ankle joints revealed inhibition of arthritis when the data were analyzed as area under the curve. Rats treated with rhuIL-1ra alone had 6% inhibition of swelling while those given 0.075 mg/kg methotrexate had a 45% decrease in ankle joint diameter over time. In contrast, those given the combination therapy had 78% inhibition of arthritis. Statistically significant combination benefit on area under the curve inhibition of ankle diameter was seen in rats given 0.048 but not 0.06 mg/kg methotrexate.

Histologic evaluation of ankle joints from rats treated with rhuIL-1ra demonstrated 8% inhibition of inflammation and 53% inhibition of bone resorption. Treatment with methotrexate resulted in dose responsive - significant inhibition of these parameters at 0.075 (44% inhibition of inflammation and 58% inhibition of bone resorption) or 0.06 (26% inhibition of inflammation and 55% inhibition of bone resorption) but not 0.048 mg/kg (8% inhibition of inflammation and 11% inhibition of bone resorption). Combination benefit was most striking in rats given the 0.075 mg/kg dose of methotrexate in combination with rhuIL-1ra. In these rats, inflammation was inhibited by 85% and bone resorption was decreased by 97%. These differences were significantly increased over those seen in either - treatment alone for both parameters. Similar combination benefit was also seen at 0.06 mg/kg dose of methotrexate in combination with rhuIL-1ra (44% inhibition of inflammation and 84% inhibition of bone resorption)-and with 0.048 mg/kg dose of methotrexate in combination with rhuIL-1ra (23% inhibition of inflammation and 68% inhibition of bone resorption).

## Claims

1. Use of methotrexate in the preparation of a medicament for treating an acute or chronic inflammatory disease in a patient in need thereof in combination with the prior, concurrent or post-administration of an interleukin-1 (IL-1) inhibitor.

2. Use of an interleukin-1 (IL-1) inhibitor in the preparation of a medicament for treating an acute or chronic inflammatory disease in a patient in need thereof in combination with the prior, concurrent, or post-administration of methotrexate.

3. A use according to any one of claims 1 to 2 wherein said methotrexate is administered orally.

4. A use according to any one of claims 1 to 3 wherein said IL-1 inhibitor is an IL-1 receptor antagonist (IL-1ra).

5. A use according to claim 4, wherein said IL-1ra is fused with all or part of a constant domain of a heavy or light chain of human immunoglobulin at the amino terminus thereof.

6. A use according to claim 5, wherein said IL-1ra is fused with all or part of a constant domain of an IgG1 heavy chain at the amino terminus thereof.

7. A use according to claim 5, wherein said constant domain comprises all domains except the first domain of the constant region of such heavy chain.

8. A use according to claim 7, wherein said heavy chain is selected from the group consisting of the heavy chain of IgG, IgA, IgM or IgE.

9. A use according to claim 8, wherein said IgG is IgG1.

10. A use according to any one of claims 4 to 9, wherein said IL-1ra comprises a sequence which is at least about 80% homologous to the amino acid sequence of SEQ ID NO:2.

11. A use according to any one of claims 4 to 9, wherein said IL-1ra comprises a sequence which is at least about 90% homologous to the amino acid sequence of SEQ ID NO:2.

12. A use according to any one of claims 4 to 9, wherein said IL-1ra comprises a sequence which is at least about 95% homologous to the amino acid sequence of SEQ ID NO:2.

13. A use according to any one of claims 4 to 9, wherein said IL-1ra comprises a sequence which is at least about 99% homologous to the amino acid sequence of SEQ m NO:2.

14. A use according to any one of claims 4 to 9, wherein said IL-1ra comprises the amino acid sequence of SEQ ID NO:2 or a biologically active deletion variant thereof.

15. A use according to claim 14, wherein said IL-1ra comprises an N-terminal or C-terminal deletion of SEQ m NO:2.

16. A use according to any one of claims 4 to 9, wherein said IL-1ra comprises an amino acid sequence having a sequence of SEQ ID NO:2.

17. A use according to any one of claims 4 to 9, wherein said IL-1ra consists of an amino acid sequence having a sequence of SEQ ID NO:2.

18. A use according to any one of claims 10 to 17, with the initial amino acid in SEQ ID NO:2 being Mn wherein n equals 1.

19. A use according to any one of claims 4 to 18, wherein said IL-1ra is non-glycosylated.

20. A use according to any one of claims 4 to 18, wherein said IL-1ra is glycosylated.

21. A use according to any one of claims 4 to 20, wherein said IL-1ra is produced by recombinant DNA methods.

22. A use according to any one of claims 1 to 21, wherein said inflammatory disease is an inflammatory condition of a joint.

23. A use according to any one of claims 1 to 21, wherein said inflammatory disease is rheumatoid arthritis.

## Patentansprüche

1. Verwendung von Methotrexat bei der Herstellung eines Medikaments zum Behandeln einer akuten oder chronischen entzündlichen Erkrankung bei einem Patienten mit Bedarf dafür, in Kombination mit der vorherigen, gleichzeitigen oder anschließenden Verabreichung eines Inhibitors von Interleukin-1 (IL-1).

2. Verwendung eines Inhibitors von Interleukin-1 (IL-1) bei der Herstellung eines Medikaments zum Behandeln einer akuten oder chronischen entzündlichen Erkrankung bei einem Patienten mit Bedarf dafür, in Kombination mit der vorherigen, gleichzeitigen oder anschließenden Verabreichung von Methotrexat.

3. Verwendung gemäß einem der Ansprüche 1 bis 2, wobei das Methotrexat oral verabreicht wird.

4. Verwendung gemäß einem der Ansprüche 1 bis 3, wobei es sich bei dem Inhibitor von IL-1 um einen IL-1-Rezeptor-Antagonisten (IL-1ra) handelt.

5. Verwendung gemäß Anspruch 4, wobei der IL-1ra mit der gesamten oder einem Teil der konstanten Domäne einer schweren oder leichten Kette von menschlichem Immunglobulin an deren Aminoterminus fusioniert ist.

6. Verwendung gemäß Anspruch 5, wobei der IL-1ra mit der gesamten oder einem Teil der konstanten Domäne einer schweren IgG1-Kette an deren Aminoterminus fusioniert ist.

7. Verwendung gemäß Anspruch 5, wobei die konstante Domäne alle Domänen außer der ersten Domäne der konstanten Region einer derartigen schweren Kette umfasst.

8. Verwendung gemäß Anspruch 7, wobei die schwere Kette aus der Gruppe, bestehend aus der schweren Kette von IgG, IgA, IgM oder IgE, ausgewählt ist.

9. Verwendung gemäß Anspruch 8, wobei es sich bei dem IgG um IgG1 handelt.

10. Verwendung gemäß einem der Ansprüche 4 bis 9, wobei der IL-1ra eine Sequenz umfasst, die zu der Aminosäuresequenz der SEQ ID NO: 2 mindestens etwa 80% homolog ist.

11. Verwendung gemäß einem der Ansprüche 4 bis 9, wobei der IL-1 ra eine Sequenz umfasst, die zu der Aminosäuresequenz der SEQ ID NO: 2 mindestens etwa 90% homolog ist.

12. Verwendung gemäß einem der Ansprüche 4 bis 9, wobei der IL-1ra eine Sequenz umfasst, die zu der Aminosäuresequenz der SEQ ID NO: 2 mindestens etwa 95% homolog ist.

13. Verwendung gemäß einem der Ansprüche 4 bis 9, wobei der IL-1ra eine Sequenz umfasst, die zu der Aminosäuresequenz der SEQ ID NO: 2 mindestens etwa 99% homolog ist.

14. Verwendung gemäß einem der Ansprüche 4 bis 9, wobei der IL-1ra die Aminosäuresequenz der SEQ ID NO: 2 oder einer biologisch aktiven Deletionsvariante davon umfasst.

15. Verwendung gemäß Anspruch 14, wobei der IL-1ra eine N-terminale oder C-terminale Deletion der SEQ ID NO: 2 umfasst.

16. Verwendung gemäß einem der Ansprüche 4 bis 9, wobei der IL-1ra eine Aminosäuresequenz mit einer Sequenz der SEQ ID NO: 2 umfasst.

17. Verwendung gemäß einem der Ansprüche 4 bis 9, wobei der IL-1ra aus einer Aminosäuresequenz mit einer Sequenz der SEQ ID NO: 2 besteht.

18. Verwendung gemäß einem der Ansprüche 10 bis 17, wobei die anfängliche Aminosäure in der SEQ ID NO: 2 Mn ist, wobei n gleich 1 ist.

19. Verwendung gemäß einem der Ansprüche 4 bis 18, wobei der IL-1ra nicht glycosyliert ist.

20. Verwendung gemäß einem der Ansprüche 4 bis 18, wobei der IL-1ra glycosyliert ist.

21. Verwendung gemäß einem der Ansprüche 4 bis 20, wobei der IL-1ra durch rekombinante DNA-Verfahren hergestellt wird.

22. Verwendung gemäß einem der Ansprüche 1 bis 21, wobei es sich bei der entzündlichen Erkrankung um ein entzündliches Gelenkleiden handelt.

23. Verwendung gemäß einem der Ansprüche 1 bis 21, wobei es sich bei der entzündlichen Erkrankung um rheumatoide Arthritis handelt.

## Revendications

1. Utilisation de méthotrexate dans la préparation d'un médicament destiné au traitement d'une maladie inflammatoire aiguë ou chronique chez un patient en ayant besoin en combinaison avec l'administration antérieure, simultanée ou postérieure d'un inhibiteur d'interleukine-1 (IL-1).

2. Utilisation d'un inhibiteur d'interleukine-1 (IL-1) dans la préparation d'un médicament destiné au traitement d'une maladie inflammatoire aiguë ou chronique chez un patient en ayant besoin en combinaison avec l'administration antérieure, simultanée ou postérieure de méthotrexate.

3. Utilisation selon l'une quelconque des revendications 1 à 2, dans laquelle ledit méthotrexate est administré par voie orale.

4. Utilisation selon l'une quelconque des revendications 1 à 3, dans laquelle ledit inhibiteur d'IL-1 est un antagoniste du récepteur de l'IL-1 (IL-1ra).

5. Utilisation selon la revendication 4, dans laquelle ledit IL-1ra est fusionné avec la totalité ou une partie d'un domaine constant d'une chaîne lourde ou légère d'immunoglobuline humaine au niveau de son N-terminal.

6. Utilisation selon la revendication 5, dans laquelle ledit IL-1ra est fusionné avec la totalité ou une partie d'un domaine constant d'une chaîne lourde d'IgG1 au niveau de son N-terminal.

7. Utilisation selon la revendication 5, dans laquelle ledit domaine constant comprend tous les domaines sauf le premier domaine de la région constante d'une telle chaîne lourde.

8. Utilisation selon la revendication 7, dans laquelle ladite chaîne lourde est choisie dans le groupe constitué par la chaîne lourde d'un IgG, IgA, IgM ou IgE.

9. Utilisation selon la revendication 8, dans laquelle ladite IgG est l'IgGl.

10. Utilisation selon l'une quelconque des revendications 4 à 9, dans laquelle ledit IL-1ra comprend une séquence qui a une homologie d'au moins environ 80 % avec la séquence d'acides aminés de SÉQ ID NO : 2.

11. Utilisation selon l'une quelconque des revendications 4 à 9, dans laquelle ledit IL-1ra comprend une séquence qui a une homologie d'au moins environ 90 % avec la séquence d'acides aminés de SÉQ ID NO : 2.

12. Utilisation selon l'une quelconque des revendications 4 à 9, dans laquelle ledit IL-1ra comprend une séquence qui a une homologie d'au moins environ 95 % avec la séquence d'acides aminés de SÉQ ID NO : 2.

13. Utilisation selon l'une quelconque des revendications 4 à 9, dans laquelle ledit IL-1ra comprend une séquence qui a une homologie d'au moins environ 99 % avec la séquence d'acides aminés de SÉQ ID NO : 2.

14. Utilisation selon l'une quelconque des revendications 4 à 9, dans laquelle ledit IL-1ra comprend la séquence d'acides aminés de SÉQ ID NO : 2 ou un variant de délétion biologiquement actif de celle-ci.

15. Utilisation selon la revendication 14, dans laquelle ledit IL-1ra comprend une délétion N-terminale ou C-terminale de SÉQ ID NO : 2.

16. Utilisation selon l'une quelconque des revendications 4 à 9, dans laquelle ledit IL-1ra comprend une séquence d'acides aminés ayant une séquence de SÉQ ID NO : 2.

17. Utilisation selon l'une quelconque des revendications 4 à 9, dans laquelle ledit IL-1ra consiste en une séquence d'acides aminés ayant une séquence de SÉQ ID NO : 2.

18. Utilisation selon l'une quelconque des revendications 10 à 17, l'acide aminé initial de SÉQ ID NO : 2 étant Mn, où n est égal à 1.

19. Utilisation selon l'une quelconque des revendications 4 à 18, dans laquelle ledit IL-1ra est non glycosylé.

20. Utilisation selon l'une quelconque des revendications 4 à 18, dans laquelle ledit IL-1ra est glycosylé.

21. Utilisation selon l'une quelconque des revendications 4 à 20, dans laquelle ledit IL-1ra est produit par des procédés de recombinaison de l'ADN.

22. Utilisation selon l'une quelconque des revendications 1 à 21, dans laquelle ladite maladie inflammatoire est un état inflammatoire d'une articulation.

23. Utilisation selon l'une quelconque des revendications 1 à 21, dans laquelle ladite maladie inflammatoire est la polyarthrite rhumatoïde.


### SEQUENCE LISTING

(1) GENERAL INFORMATION:
   (i) APPLICANT: Amgen Inc.
   (ii) TITLE OF INVENTION: COMBINATION THERAPY USING AN IL-1 INHIBITOR FOR TREATING IL-1 MEDIATED DISEASES
   (iii) NUMBER OF SEQUENCES: 2
   (iv) CORRESPONDENCE ADDRESS:
      (A) ADDRESSEE: Amgen Inc.
      (B) STREET: 1840 DeHavilland Drive
      (C) CITY: Thousand Oaks
      (D) STATE: CA
      (E) COUNTRY: US
      (F) ZIP: 91320-1789
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: NOT YET KNOWN
      (B) FILING DATE: 08-DEC-1997
      (C) CLASSIFICATION:
   (vii) PRIOR APPLICATION DATA:
      (A) APPLICATION NUMBER: US 60/032,790 (B) FILING DATE: 06-DEC-1996
   (vii) PRIOR APPLICATION DATA:
      (A) APPLICATION NUMBER: US 60/036,353
      (B) FILING DATE: 23-JAN-1997
   (vii) PRIOR APPLICATION DATA:
      (A) APPLICATION NUMBER: US 60/039,311
      (B) FILING DATE: 07-FEB-1997
   (vii) PRIOR APPLICATION DATA:
      (A) APPLICATION NUMBER: US 60/052,025
      (B) FILING DATE: 09-JUL-1997
   (viii) ATTORNEY/AGENT INFORMATION:
      (A) NAME: Zindrick, Thomas D.
      (C) REFERENCE/DOCKET NUMBER: A-398D
(2) INFORMATION FOR SEQ ID NO:1:
   (i) SEQUENCE CHARACTERISTICS:
      (A) LENGTH: 462 base pairs
      (B) TYPE: nucleic acid
      (C) STRANDEDNESS: unknown
      (D) TOPOLOGY: unknown
   (ii) MOLECULE TYPE: cDNA
   (ix) FEATURE:
      (A) NAME/KEY: CDS
      (B) LOCATION: 1..462
   (xi) SEQUENCE DESCRIPTION: SEQ ID NO:1:
   ATG CGA CCC TCT GGG AGA AAA TCC AGC AAG ATG CAA GCC TTC AGA ATC      48
   Met Arg Pro Ser Gly Arg Lys Ser Ser Lys Met Gln Ala Phe Arg Ile
    1               5                  10                  15
   TGG GAT GTT AAC CAG AAG ACC TTC TAT CTG AGG AAC AAC CAA CTA GTT      96
   Trp Asp Val Asn Gln Lys Thr Phe Tyr Leu Arg Asn Asn Gln Leu Val
               20                  25                  30
   GCT GGA TAC TTG CAA GGA CCA AAT GTC AAT TTA GAA GAA AAG ATA GAT     144
   Ala Gly Tyr Leu Gln Gly Pro Asn Val Asn Leu Glu Glu Lys Ile Asp
           35                  40                  45
   GTG GTA CCC ATT GAG CCT CAT GCT CTG TTC TTG GGA ATC CAT GGA GGG     192
   Val Val Pro Ile Glu Pro His Ala Leu Phe Leu Gly Ile His Gly Gly
       50                  55                  60
   AAG ATG TGC CTG TCC TGT GTC AAG TCT GGT GAT GAG ACC AGA CTC CAG     240
   Lys Met Cys Leu Ser Cys Val Lys Ser Gly Asp Glu Thr Arg Leu Gln
   65                  70                  75                  80
   CTG GAG GCA GTT AAC ATC ACT GAC CTG AGC GAG AAC AGA AAG CAG GAC     288
   Leu Glu Ala Val Asn Ile Thr Asp Leu Ser Glu Asn Arg Lys Gln Asp
                   85                  90                  95
   AAG CGC TTC GCC TTC ATC CGC TCA GAC AGT GGC CCC ACC ACC AGT TTT     336
   Lys Arg Phe Ala Phe Ile Arg Ser Asp Ser Gly Pro Thr Thr Ser Phe
               100                 105                 110
   GAG TCT GCC GCC TGC CCC GGT TGG TTC CTC TGC ACA GCG ATG GAA GCT     384
   Glu Ser Ala Ala Cys Pro Gly Trp Phe Leu Cys Thr Ala Met Glu Ala
           115                 120                 125
   GAC CAG CCC GTC AGC CTC ACC AAT ATG CCT GAC GAA GGC GTC ATG GTC     432
   Asp Gln Pro Val Ser Leu Thr Asn Met Pro Asp Glu Gly Val Met Val
       130                 135                 140
   ACC AAA TTC TAC TTC CAG GAG GAC GAG TAG                             462
   Thr Lys Phe Tyr Phe Gln Glu Asp Glu  *
   145                 150
(2) INFORMATION FOR SEQ ID NO:2:
   (i) SEQUENCE CHARACTERISTICS:
      (A) LENGTH: 154 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Arg Pro Ser Gly Arg Lys Ser Ser Lys Met Gln Ala Phe Arg Ile
    1               5                  10                  15
   Trp Asp Val Asn Gln Lys Thr Phe Tyr Leu Arg Asn Asn Gln Leu Val
               20                  25                  30
   Ala Gly Tyr Leu Gln Gly Pro Asn Val Asn Leu Glu Glu Lys Ile Asp
           35                  40                  45
   Val Val Pro Ile Glu Pro His Ala Leu Phe Leu Gly Ile His Gly Gly
       50                  55                  60
   Lys Met Cys Leu Ser Cys Val Lys Ser Gly Asp Glu Thr Arg Leu Gln
   65                  70                  75                  80
   Leu Glu Ala Val Asn Ile Thr Asp Leu Ser Glu Asn Arg Lys Gln Asp
                   85                  90                  95
   Lys Arg Phe Ala Phe Ile Arg Ser Asp Ser Gly Pro Thr Thr Ser Phe
               100                 105                 110
   Glu Ser Ala Ala Cys Pro Gly Trp Phe Leu Cys Thr Ala Met Glu Ala
           115                 120                 125
   Asp Gln Pro Val Ser Leu Thr Asn Met Pro Asp Glu Gly Val Met Val
       130                 135                 140
   Thr Lys Phe Tyr Phe Gln Glu Asp Glu  *
   145                 150